# EUROPÄISCHE PATENTANMELDUNG

(11) **EP 1 891 914 A1**
(43) Veröffentlichungstag der Anmeldung: **27.02.2008**
(21) Anmeldenummer: 07016738.2
(22) Anmeldetag: 27.08.2007
(51) Int. Cl.: A61F 2/84

(54) **Katheter-Stent-Vorrichtung**

(30) Priorität: 25.08.2006 DE 102006039823
(71) Anmelder: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz Truckenmüller Lucht

(57) **Zusammenfassung**

Die Erfindung betrifft eine Katheter-Stent-Vorrichtung nach dem Patent DE 10 2005 008 682 zur Applikation und Implantation eines StenLs zur Behandlung von Körpergefäßen oder Körperhohlorganen.

Dabei liegt der Erfindung die Aufgabe zugrunde, eine verbesserte Katheter-Stent-Vorrichtung zu schaffen, die das bislang in Verbindung mit der Freisetzung einer Embolisationsspirale aus einem Katheter vorbekannte hydraulische Prinzip nun in Verbindung mit einem selbstexpandierenden Stent einsetzt.

Dies gelingt, indem ein Stent und ein diesen Stent umgebender Katheter mittels einer Druckdifferenz zwischen einem proximalen - außerhalb des Körpers gelegenen - und einem distalen - im Körper gelegenen - Ende des Katheter herrschenden Druckdifferenz in Relation zueinander beweglich sind, wobei diese Druckdifferenz mittels eines Druckmediums, bestehend aus einer Flüssigkeit oder aus einem Gas, aufbringbar und hierdurch der Stent zielgerecht implantierbar ist.

## Beschreibung

Die Erfindung betrifft eine Katheter-Stent-Vorrichtung nach dem Patent DE 10 2005 008 682 zur Applikation und Implantation eines Stents zur Behandlung von Körpergefäßen oder Körperhohlorganen.

Stents haben sich als Mittel zur Behandlung von pathologisch veränderten Körpergefäßen und Hohlorganen durchgesetzt und neue Behandlungsmöglichkeiten eröffnet. Im Vergleich mit ballonexpandierbaren Stents weisen selbstexpandierende Stents einige technische Vorteile auf wie z.B. einen kleineren Einführdurchmesser und stärkere Flexibilität und Elastizität des Stents.

Die zur Zeit angewendeten Systeme zur Freisetzung eines selbstexpandierenden Stents bestehen aus einer Hülle, in denen der Stent im nicht expandierten Zustand gehalten wird und einem Pushersystem, das den Stent aus der Hülle in das zu behandelnde Gefäß treibt. Dieser Pusher enthält einen Kanal für den Angiographie-Führungsdraht. Die Nachteile dieses Systems liegen darin, dass der Pusher zum Austreiben des Stents mit einer hohen Kraft belastet werden muss, um den Stent herauszutreiben bzw. die Hülle zurück zu ziehen.

Deswegen ist der Pusher meistens aus relativ starrem Material gefertigt. Beim Heraustreiben des Stents entsteht eine große Reibung nicht nur zwischen Stent und Hülle, sondern zusätzlich auch zwischen Pusher und Hülle. Deswegen ist dieses System schlecht für geschlängelte Gefäße geeignet. Es kommt vor, dass das eingeführte Katheter-Stent-Assembly im Patienten versagt und dass sich der Pusher nicht vorschieben lässt oder die Hülle nicht zurück ziehen lässt, besonders in Fällen von Implantation gastrointestinaler Stents. Selbst expandierende Stents werden deswegen aus besagten technischen Gründen nicht für Koronararterien verwendet.

Ein weiteres technisches Problem mit zurzeit verfügbaren Stentapplikationssystemen ist der sogenannte "Jump-Effekt", der beobachtet werden kann, wenn der nach fast vollständiger Freisetzung noch im Applikationssystem verbliebene Restanteil des Stents die äußere Umhüllung verlässt, und zwar sprungartig, da beim Austritt des Stents aus der ihn umgebenden Hülle die Energien innerhalb des Applikationssystems, bestehend aus Stauchung des Pushers und Zug auf die Hülle, plötzlich freigesetzt werden; dieses am Ende des Freisetzungsvorganges auftretende unkontrollierte Freisetzen des letzten Anteils des Stents kann zur Fehlplatzierung des Stents und/oder Verletzungen der inneren Gefäßwand führen und somit den technischen sowie den klinischen Erfolg der Stenttherapie erheblich mindern.

Zur Lösung der erwähnten Probleme schlägt schon der Stand der Technik vor, die Kraft zum Heraustreiben des Stents aus dem Katheter (Hülle) nicht durch einen Pusher zu übertragen, sondern durch eine Flüssigkeits- oder Gassäule. Anwendbar wäre das Prinzip der Hydraulik oder der Pneumatik, d.h. die Kraft zum Heraustreiben des Stents aus der Hülle wird durch eine Flüssigkeits- oder Gassäule übertragen. Das hydraulische Prinzip gewährleistet dabei eine geschmeidigere und besser kontrollierbare und dosierbare Kraftübertragung auf den Stent, der aus der ihn umgebenden Hülle freigesetzt werden soll.

Das Prinzip der Hydraulik wird ansatzweise bereits im Zusammenhang mit einem handelsüblichen Produkt, der Embolisationsspirale, angewendet: eine in einem Katheter befindliche fadenartige Drahtstruktur wird durch Einspülen von Flüssigkeit aus dem Katheter herausgeschwemmt und so in das zu behandelnde Gefäß gebracht, wo es sich zu einem Knäuel entfaltet und das Gefäßlumen verschließt. Da es sich bei der Embolisationsspirale jedoch um eine kleine, dünne und schwache fadenartige Struktur handelt, die eine nur geringe radiäre Expansionskraft aufweist, wird nur geringe Kraft benötigt, die Spirale aus dem Katheter hinaus zu befördern, da keine nennenswerten Reibungskräfte entstehen. Bei der besagten Anwendung ist es nicht nötig, dem Knäuel für die Implantation eine bestimmte Form zu geben und dieses kontrolliert und schubweise aus dem Katheter heraustreten zu lassen. Die Kraftübertragung durch einen Kolben wurde bislang nicht angewendet. Die Anwendung eines solchen garantiert jedoch eine genauere Platzierung.

Die Embolisationsspirale steht im Gegensatz zu selbstexpandierenden Stents, die eine sehr starke radiäre Kraft aufweisen und damit im Katheter hohe Reibungskräfte verursachen.

Der Erfindung liegt die Aufgabe zugrunde, eine verbesserte Katheter-Stent-Vorrichtung zu schaffen, die das vorstehend erläuterte hydraulische Prinzip in Verbindung mit einem selbstexpandierenden Stent einsetzt. Hierbei besteht das Problem, dass derartigen Stents eine starke Radialkraft innewohnt, die den Vortrieb des Stent in einem Katheter erschweren und hohe Reibungskräfte verursachen.

Die Lösung dieser Aufgabe gelingt durch eine Vorrichtung mit den Merkmalen des Hauptanspruchs. Vorteilhafte Ausgestaltungen können den Unteransprüchen entnommen werden.

Die Lösung dieser Aufgabe gelingt dadurch, dass der Stent und der Katheter zueinander relativ beweglich ausgebildet sind und der Stent einem vorgegeben Druckgefälle folgend implantiert wird. Der zu platzierende Stent ist ein dabei z.B. ein Spiralstent, z.B. ein Doppelcoil-Stent, ein Mäander-Stent, oder Helix-Stent, die vor der Freisetzung in ihrem gestreckten Zustand das Lumen des Katheters nicht vollständig ausfüllen. Das Druckmedium, z.B. Flüssigkeit oder Gas, fließt teilweise am Coil-Stent vorbei und dient damit gleichzeitig als Gleitmittel zur Reduktion der Reibungskräfte zwischen Stent und Katheter.

Die Spitze des Coil-Stents, also des Drahtes, ist wie ein Kolben ausgebildet. Der Kolben hat einen so großen Durchmesser, dass er das Innenlumen des Katheters ausfüllt. Flüssigkeit, die vom proximalen Ende in den Katheter hineingedrückt wird, trifft auf den Kolben und presst ihn aus dem Katheter hinaus.

Der Kolben dichtet den Zwischenraum (Spalt) so ab, dass das Druckmedium nicht zwischen Kolbenoberfläche und Katheterinnenwand vorbeifließen kann. Der am distalen Ende des Stents angebrachte Kolben zieht dann den restlichen Stent hinter sich her und im Ergebnis aus dem Katheter heraus. Somit wird die Helix-Wendel nicht proximalseitig gestaucht, wodurch geringe Reibungsenergie entsteht.

Hat der Kolben den Katheter verlassen, könnte allerdings die auf den Stent einwirkende Kraft zu gering sein, um den restlichen Stent vollständig aus dem Katheter freizusetzen. Deshalb können noch weitere kolbenförmige Erweiterungen am Stentdraht, am proximalen Ende des Stents oder in der Stentmitte, angebracht sein, die dann ihrerseits als zusätzliche Kraftüberträger wirken.

Diese zusätzlichen kolbenförmigen Erweiterungen könnten einen etwas kleineren Durchmesser haben als der Innendurchmesser des umgebenden Katheters oder mit Löchern ausgestattet sein, womit eine Leckage entsteht, damit zu Beginn der Freisetzung die Flüssigkeit bis an den ersten Kolben vordringen kann.

In einer anderen Ausführung könnten die zusätzlichen kolbenförmigen Erweiterungen gleich groß ausgebildet sein und jeweils das Lumen des Katheters ausfüllen.

Der am Stentdraht angebrachte Kolben und/oder die kolbenförmigen Erweiterungen können jeweils aus biologisch abbaubarem Material gefertigt sein und lösen sich im Blut auf, damit sie den Blutfluss nicht behindern.

Zumindest der vorderste Kolben legt sich nach Implantation des Stents flach an die Gefäßwand an, so dass keine Turbulenzen entstehen.

Alternativ kann der Stent auch mittels eines separaten Kolbens der über das Druckmedium auf den Stent einwirkt innerhalb des Katheters vorangetrieben werden.

Der bewegliche Kolben weist einen zentralen Kanal auf, durch den der Führungsdraht geführt ist. Der Durchmesser des Kanals ist so gewählt, dass der Führungsdraht den Kanal vollständig ausfüllt und abdichtet, damit kein Druckverlust entstehen kann. In diesem Fall verfügt der bewegliche Kolben mit Vorteil über zusätzliche Abdichtungen, z.B. Dichtungsringe.

Bei kleinerem Kolbendurchmesser kann der Kolben in seinen Dimensionen so ausgelegt sein, dass der Kolben sich zusammen mit dem im Katheter befindlichen Drahtanteil innerhalb des umgebenden Katheters um seine Längsachse drehen kann, während der gestreckte Helixdrahtstent heraus getrieben wird. Der bereits ins Gefäß implantierte Anteil des Helixstents bewegt sich dann nicht mehr, sondern nur noch der noch im Katheter befindliche Anteil des Stents. Hierdurch wird eine regelrechte Platzierung des Stent in seiner vorgegebenen Form erreicht. Dabei wird eine Schädigung der Gefäßwand vermieden, die durch einen sich im Gefäß bewegenden Stentanteil hervorgerufen werden könnte. Ebenso vermieden wird eine ungewünschte ungünstige Verwerfung der Helixform.

Im Falle von herkömmlichen selbst expandierenden, geschlitzten Rohrstents, z.B. aus Nitinol, oder geflochtenen und gestrickten schlauchförmigen Stents wird der Katheter in seinem Inneren mit einem Kolben ausgestattet, der den Stent durch den Katheter drückt und aus dem Katheter herausdrückt. Der Kolben verbleibt im Katheter, dies wird durch eine Sicherung, z.B. einen Faden gewährleistet, der im Katheter verankert ist.

Um zu verhindern, dass der endständige Kolben nach Freisetzung des Stents den Katheter verlässt, befindet sich in der Wand des Katheters ein Seitloch. Solange sich der Stent, oder Anteile des Stents, noch im Katheter befinden, ist das Seitloch durch den Kolben verschlossen. Nach Freisetzung des Stents befindet sich der Kolben an der Katheterspitze und das Seitloch ist offen, die als Druckmedium dienende einströmende Flüssigkeit kann nun durch das Seitloch austreten. Damit verringert sich der Druck auf den Kolben, so dass der Kolben sich nicht weiter bewegt. Der Abstand des Seitlochs von der Katheterspitze muss somit gleich groß sein wie die Länge des Kolbens.

Die vordere Spitze des Stents ist wie eine Kugel ausgebildet, die von einem Gabeldraht aufgenommen wird. Der Gabeldraht ist fest mit einem Schiebekolben verbunden, die zusammen einen Stentträger (Carrier) bilden; der Schiebekolben befindet sich am proximalen Ende des Stents. Ein Druckmedium, etwa eine Flüssigkeit, die vom proximalseitig in den Katheter hineingedrückt wird, trifft dann auf den Schiebekolben und bewegt den am Gabeldraht gehaltenen Stent zusammen mit diesem Gabeldraht aus dem Katheter hinaus. Der Stent löst sich vom Gabeldraht und der Gabeldraht wird dann zusammen mit dem Katheter und dem innenliegenden Schiebekolben entfernt.

Der Schiebekolben insgesamt ist flexibel und biegsam, damit das System durch geschlängelt verlaufende Arterien geschoben werden kann und auch in einer Arterienkurve freigesetzt werden kann.

Bei einer weiteren Ausführung stellt der rohr- oder schlauchförmige Stent in seinem kleinen Durchmesser im Katheter den Kolben selbst dar. Die Öffnung des rohrförmigen Stents im kleinen Durchmesser wird durch den Führungsdraht so ausgefüllt, dass ein Druckverlust nicht entstehen kann, wenn der Stent herausgepresst wird. Die distale Spitze des Katheters ist elastisch und konisch ausgebildet, so dass die Stufe zwischen dem Führungsdraht und dem den Stent enthaltenden Katheter ausgeglichen wird. Beim Heraustreten des Stents wird der Konus weiter und erlaubt die Passage ohne Druckabfall.

Bei einer weiteren Ausführung wird die Lücke zwischen Führungsdraht und Lumen des zusammengefalteten Stents durch eine biologisch abbaubare Substanz ausgefüllt. Auch die Lücken zwischen den einzelnen Stentstreben am Hinterende des Stents sind so mit dem Polymer ausgefüllt, dass das so verdichtete Stentende die Wirkung eines Kolbens bekommt. Beim Entfalten des Stents im Gefäß reißt diese polymerartige Substanz auf und gibt den Stent vollkommen frei

In dem Katheter liegt am distalen Ende ein länglicher Kolben, der mit einer konischen Spitze versehen ist. Durch die Spitze führt zunächst koaxial ein Kanal, der dann schräg zur Außenfläche des Kolbens führt und den Führungsdraht aufnimmt. Das dargestellte System entspricht einem Fast-Exchange-System. Am distalen Anteil dieses Kolbens findet sich ein Träger mit einer Einkerbung (Stent-Bett), in der der Stent im Low-profile-Status eingelegt wird und durch die Hülle des Einführungskatheters in seinem kleinen Durchmesser gehalten wird, der Stent ist elastisch selbstexpandierend. Der Einführungskatheter hat einen länglichen Schlitz. Dieser Schlitz liegt über der Seitöffnung des Kolbens. Der Führungsdraht wird vor der Einführung des Katheter-Stent-Assemblies in die distale Öffnung des Besteckes eingeführt und retrograd durch den Kolben geschoben, bis er aus der Seitöffnung des Kolbens sowie aus dem darüber liegenden Längsschlitz des Katheters heraus ragt. Der Katheter wird in diesem Status in ein Hohlgefäß, z.B. Arterie, in den Körper eingebracht, bis der nicht entfaltete Stent im Bereich der zu behandelnden Stelle liegt. Dann erfolgt die Druckbeaufschlagung mit dem Druckmedium im Katheter, wodurch der Kolben vorwärts geschoben wird.

In einem anderen Fall bewirkt die Druckbeaufschlagung, dass der Kolben in seiner Position verbleibt, während der Katheter zurückgezogen wird. Es muss also beim Heraustreiben des Kolbens der Katheter im gleichen Maße zurückgezogen werden, um mit dem sich expandierenden Stent die zu behandelnde Stelle in der Arterie zu treffen.

Bei der Bewegung des Kolbens gegenüber dem Katheter rutscht der Kolben, den Führungsdraht beinhaltend, nach distal, wobei sich der Führungsdraht ebenfalls in dem länglichen Schlitz nach distal bewegt.

So wird der Stent dann zur Expansion freigegeben. Dies ist der Fall, wenn das distale Ende des Katheters das Bett des Stents nicht mehr bedeckt. Ein Herausrutschen des Kolbens wird dadurch verhindert, dass der Draht beim Heraustreten aus dem seitlichen Loch des Kolbens ein Weiterrutschen des Kolbens versperrt.

Ein solcher Sicherungs-Effekt, der ein Austreten des Kolbens aus dem Katheter verhindert, kann auch durch ein Seitloch im Katheter selbst erzielt werden. Wenn der Kolben nach vorne gerutscht ist, öffnet sich das Seitloch und der Druck im System wird abgebaut.

In anderer Ausführung ist eine doppellumige Katheter-Stent-Vorrichtung ausgebildet. Der Katheter umfasst zwei separate Lumina, ein engeres und ein weiteres Lumen. Vor Einführung des Stents in das zu behandelnde Körperhohlgefäß oder -organ und zwar vor Einführung des Führungsdrahtes in den Katheter wird die Vorrichtung zunächst entlüftet, und zwar über das engere Lumen. Das engere Lumen dient später auch zur Druckbeaufschlagung der Vorrichtung. Der Vorteil des engeren Lumens besteht darin, dass es zumeist starrwandiger ist, da die Wandspannung hier geringer als bei einem weiten Lumen. Mittels eines vergleichsweise starren Lumens kann der Druck besser dosiert werden, weil sich die Wände des Lumens mit erhöhtem Druck weniger stark aufdehnen.

Zur Entlüftung wird die Vorrichtung gespült, um verbleibende Luft heraus zu befördern. Hierzu läuft das Druckmedium zunächst von proximal außerhalb des Katheters über eine Spritze durch das engere Lumen in eine Hauptdruckkammer. Die Dichtungen sind jetzt, wo noch kein Führungsdraht eingeführt ist, offen, so dass die Luft aus der Druckkammer über das nicht schließende Ventil entweichen kann, und zwar nach proximalwärts. Die Spülflüssigkeit tritt aus dem Stentträger-System aus, und zwar aus seiner Spitze.

Nach Austritt der Spülflüssigkeit wird die Spitze des Systems verschlossen, z.B. mit dem Finger, bis die Flüssigkeit dann über das zweite Ventil, das weiter proximalwärts gelegen ist, aus dem proximalen Ende des Katheters, ausgetreten ist. Daraufhin wird der Verschlusshahn am proximalen Ende des engeren Lumens des Katheters verschlossen.
Der Führungsdraht wird über die Spitze des Stentträger-Systems eingebracht und durch den Katheter geführt. Erst dann wird die entlüftete Katheter-Stent-Vorrichtung in das zu behandelnde Hohlorgan eingeführt. Die Druckbeaufschlagung zwecks Vortrieb des Stentträgers zur Stentfreisetzung erfolgt ebenfalls über das engere Lumen der Vorrichtung, wobei die Druckkammer ihr Volumen vergrößert und der Stentträger aus dem umgebenden Katheter herausbefördert wird.

Das Volumen der Spritze (Pumpe) zur Druckbeaufschlagung ist mindestens so groß wie das der Druckkammer im Zustand nach Freisetzung des Stents. Im Kolbenbereich des Stentträgers sind zwei Dichtungen zur besseren Abdichtung angebracht. Die kleinere befindet sich zwischen Führungsdraht und Innenwand des Stentträgers, die zweite besteht aus einer Scheibe aus elastischem Kunststoff z.B. Silikon und dichtet den Spalt zwischen dem Führungsdraht und dem Katheter ab. Bei Biegung des Katheters wird dieser in seinem Querschnitt oval, die scheibenförmige Dichtung wird sich diesem Umstand anpassen.

Bei dem doppellumigen Katheter-Stent-System ist das kleine Lumen, welches für die Druckbeaufschlagung verwendet wird, relativ dickwandig, verglichen mit dem Lumen für den Führungsdraht. Das kleine Lumen ist also mit einer großen Menge Wandmasse umgeben. Hierdurch wird garantiert, dass sich bei Druckbeaufschlagung die Wand des kleinen Lumens nicht aufdehnen kann und somit die Freisetzung des Stents aus dem Trägersystem gut kontrolliert erfolgt.

Eine zweite Variante besteht darin, dass statt eines doppellumigen Katheters nur ein Lumen besteht. In dieses Lumen wird über ein Ventil ein Hohldraht (Röhrchen) eingeführt, durch den Luft beim Spülen mit Flüssigkeit herausgedrückt wird. Dieses System kann über einen Führungsdraht in den Körper eingebracht werden. Der Führungsdraht wird über die Öffnung des Stentträgers koaxial durch das System geführt und gelangt proximal über ein hämostatische Ventil dann nach außen. Bevor der Katheter jedoch eingeführt wird, wird das System über ein dünnes Röhrchen, das bis in den Kanal des Stentträgers reicht, gespült, wobei Spülflüssigkeit über das Röhrchen eingelaufen lassen wird. Dies geschieht z. B. durch eine kleine Injektionsspritze. Das proximale Ende wird angehoben, wobei die Luft im System aus der distalen Spitze des Stentträgers entweichen kann. Dann wird das Röhrchen über das Ventil langsam und ebenfalls unter Injektion der Flüssigkeit zurückgezogen. Der Führungsdraht kann nun in den Katheter eingebracht werden, der völlig luftfrei geworden ist. Hierbei tritt Flüssigkeit über den Pumpenansatz zurück in die Pumpe. Wenn der Führungsdraht aus dem hämostatischen Ventil herausgetreten ist, wird die hydraulische Pumpe, die ebenfalls entlüftet ist, angesetzt und durch Druck der Stentträger herausgetrieben, wobei der Katheter entsprechend zurückgezogen wird.

Wenn das System mit Druck beaufschlagt wird, gibt es zunächst ein ruckhaftes Vorangleiten des Kolbens. Dies wird durch eine besondere Kolbenform vermieden, z.B. durch einen Balg, womit die Stententladung besser dosiert werden kann. Der Balg besteht aus einem Schlauch mit quer zur Längsachse verlaufenden Falten, der als Kolben dient. Dieser Schlauch wird vom Katheter umhüllt.

Der zusammengefaltete Schlauch hat Querfalten, die im nicht aufgedehnten Zustand zusammen geschoben sind, dadurch ist der Schlauch verkürzt. Wird am Ende des Schlauches außerhalb des Patienten Flüssigkeitsdruck eingeleitet, dehnt sich der Schlauch in der Längsrichtung, wenn die andere, gegenüberliegende Öffnung durch einen Kolben verschlossen ist. Der pfropfenförmige Kolben wird dann den Stent heraus treiben.

Der Vorteil dieses Systems liegt darin, dass der zusammengefaltete und gestauchte Schlauch direkt mit dem Schlauch, der das Druckmedium befördert, verbunden ist. Hierdurch ist die Flüssigkeit in einem abgeschlossenen Raum gefangen, die Flüssigkeit kann also nicht zwischen Kolben und Zylinder oder in den Körper heraustreten. Dies wäre von Vorteil bei Nutzung eines sogenannten geschlossenen Systems. Weiterhin kann auf Abdichtungen zwischen Kolben und Katheterinnenwand sowie zwischen Kolben und Führungsdraht verzichtet werden. Auch könnte das Produkt von vorneherein luftfrei sein, also eine Entlüftung wie zuvor beschrieben, wäre nicht notwendig.

In abermals vorteilhafter Ausgestaltung kann die Katheter-Stent-Vorrichtung auch so ausgebildet sein, dass der Stent im Stent-Bett eines Hauptkatheters aufgenommen ist, wobei der Hauptkatheter und der noch komprimierte Stent in dem Stent-Bett von einem Hüllkatheter umschlossen sind. In proximaler Richtung schließt sich an das Stent-Bett wenigstens eine Druckkammer an, die ebenfalls von dem erwähnten Hüllkatheter umschlossen ist.

Der Vorteil eines solchen Hüllkatheters besteht darin, dass die Hülle den Stent nur um wenige Millimeter überragt, so dass der insoweit verursachte Implantationsnachteil vergleichsweise gering ist. Der Vorteil einer solchen Hülle besteht nun aber darin, dass die Reibung zwischen Hüllkatheter und Stent durch entsprechende Spülung des Stent-Betts, etwa mit einer Kochsalzlösung, vermindert werden kann.

Die Druckkammer ist gegenüber dem Stent-Bett mittels eines Dichtungsrings dichtend abgeschlossen, um eine separate, gezielte Druckbeaufschlagung in der Druckkammer allein vornehmen zu können.

Der Bereich der Druckkammer und des Stent-Betts sind durch wenigstens einen Außenring - jeweils mit einer geeigneten Ringdichtung versehen - innerhalb des Hüllkatheters, der den Hauptkatheter umgreift, gegeneinander strömungstechnisch abgeriegelt.

Ein Ringkolben schließt die Druckkammer proximalseitig ab.

In vorteilhafter Weiterbildung weist der Hauptkatheter im Bereich des Stent-Betts bzw. der Druckkammer jeweils eine Öffnung auf, um auf diese Weise bedarfsweise die Druckkammer und/oder das Stent-Bett spülen zu können.

Zwischen der Stent-Bett- und der Druckkammerspülöffnung ist an der Wandung des Hauptkatheters ein innerer Ringwulst als Dichtung angeformt, der flüssigkeitsdicht mit dem von dem Hauptkatheter konzentrisch umschlossenen Führungsdraht abschließt. Bei bestimmungsgemäßer Positionierung des Hauptkatheters relativ zum Führungsdraht ist somit die in Bezug zur Druckkammerspülöffnung distalseitig angeordnete Stent-Bett-Spülöffnung bei einer von der Distalseite eingebrachten Spülung strömungstechnisch abgeschlossen. Hierdurch wird in dieser Stellung ausschließlich die Druckkammer bzw. die Druckkammern beaufschlagt.

Bei einer solchen Druckbeaufschlagung der Druckkammer mit einem Druckmedium, vorzugsweise mit einer körperverträglichen Kochsalzlösung, wird ausschließlich die Druckkammer druckbeaufschlagt. Dabei ist der insoweit aufzubringende hydraulische Druck allerdings technisch begrenzt, so dass es sich bewährt hat, in der oder den Druckkammern jeweils zusätzlich wenigstens eine Druckfeder anzuordnen, die spiralförmig den Hauptkatheter umschließt, deren Kraft in proximaler Richtung auf den proximalseitig im Hüllkatheter angeordneten Ringkolben einwirkt.

In konkreter Ausgestaltung wird die Katheter-Stent-Vorrichtung vor ihrer Implantation etwa einfach mit dem Finger distalseitig verschlossen und anschließend zum Zwecke der Luftevakuierung unmittelbar vor der Implantation, vorzugsweise mittels einer Kochsalzlösung, gespült. Zu diesem Zeitpunkt ist der Führungsdraht entweder nicht eingeführt oder soweit herausgezogen, dass sowohl die Druckkammerspülöffnung, wie auch die Stent-Bettspülöffnung durch die Spülflüssigkeit erreichbar und somit eine Evakuierung etwa eingeschlossener Luft sowohl aus dem Stent-Bett, wie auch aus der Druckkammer heraus bewirkt wird. Dies ist zwingend notwendig, da die Einbringung von auch kleinsten Luftbläschen, insbesondere im Bereich der Koronar-Arterien, unter keinen Umständen tolerierbar ist, insbesondere für etwaige Behandlung der Arteria-Carotis mit Stents. Bereits kleine Luftblasen können die Hirnarterien verstopfen und somit das Hirngewebe schädigen.

Zum Zwecke der Spülung kann das distale Ende des fraglichen Katheters einfach mit dem Finger distalseitig verschlossen werden.

Die Katheter-Stent-Vorrichtung wird anschließend über den zuvor in den Patientenkörper eingebrachten Führungsdraht in den Körper eingebracht, lagerichtig positioniert und anschließend der Stent vor Ort bestimmungsgemäß implantiert. Hierzu wird, wie bereits vorstehend erläutert, die Druckkammer mittels eines Druckmediums beaufschlagt. Zur Unterstützung der in der Druckkammer zusätzlich angeordneten wenigstens einen Druckfeder wird hierbei der Hüllkatheter distalseitig relativ zum Stent und zum Stent-Bett zurückbewegt und hierdurch der Stent am Implantationsort freigegeben. Der bislang durch den Hüllkatheter eingeschnürte selbstexpandierende Stent expandiert daher bestimmungsgemäß in dem zu behandelnden Körpergefäß. Anschließend kann der Hauptkatheter mitsamt dem Führungsdraht durch den expandierten Stent in distaler Richtung zurückgezogen werden.

In alternativer Ausgestaltung kann sowohl bei der Katheterhülle zwischen einem distalen und einem proximalen Hüllabschnitt, wie auch bei dem auf dem Stent-Bett aufgenommenen Stent-Bett zwischen zwei Stentabschnitten unterschieden werden. Bei der Katheterhülle handelt es sich um eine echte Zweiteilung der Katheterhülle, die nachstehend noch näher erläutert wird. Der Stent ist aber einteilig ausgeführt, wobei allerdings die dem selbstexpandierenden Stent vorgeprägte Form der einzelnen Stentabschnitte unterschiedlich ist. Bei dem distalen Stentabschnitt handelt es sich um einen herkömmlichen, selbstexpandierenden Stent, der sich am Implantationsort nach Freisetzung von einem geringeren Lumen zu einem größeren Lumen bestimmungsgemäß aufweitet. Bei dem zweiten Stentabschnitt, der bei bestimmungsgemäßer Positionierung proximalseitig gegenüber dem anderen Stentabschnitt angeordnet ist, handelt es sich um einen Stentabschnitt, der sich bestimmungsgemäß ringflanschartig erweitert. Dies gelingt beispielsweise dadurch, dass in den Stent radiär abzweigende Stentstreben eingearbeitet sind, die sich nach Freisetzung radial nach außen aufdehnen und somit den fraglichen Ringflansch eröffnen. Beide Stentabschnitt sind dabei selbstexpandierend ausgelegt.

In dieser Ausführung ist die Druckkammer distalseitig relativ zum Stent-Bett angeformt.

Die Druckkammer besitzt auch bei dieser Ausführung eine Spülöffnung zum Hauptkatheter auf.

Der Hauptkatheter ist in vorteilhafter Ausgestaltung distalseitig verschlossen. Sobald der Hauptkatheter in den Patientenkörper eingeführt ist, wird er allerdings auch proximalwärts bestimmungsgemäß verschlossen.

Die Erfindung wird nachstehend zum vertieften Verständnis anhand einiger in der Zeichnung nur schematisch dargestellter Ausführungsbeispiele erläutert, ohne dass sich der Gegenstand der Erfindung in diesen Beispielen erschöpft.

Es zeigen:
Figur 3:
   Bei dieser Ausführung ist das distale Ende eines im Katheter 1 befindlichen gestreckten Spiralstents 2' mit einem distalen Kolben 4' versehen, der an den Durchmesser des Lumens des Patientenkatheters 1 angepasst ist. Hierdurch entsteht eine bessere Abdichtung zwischen dem Druckmedium und der Innenwand des Katheters 1, wenn das Druckmedium den Spiralstent 2' durch den Katheter 1 befördert. Durch den distalen Kolben 4' wird der Spiralstent 2' durch den Katheter 1 gezogen. Weil der Stent 2' hierbei nicht gestaucht wird, entsteht weniger Reibung.
Figur 4:

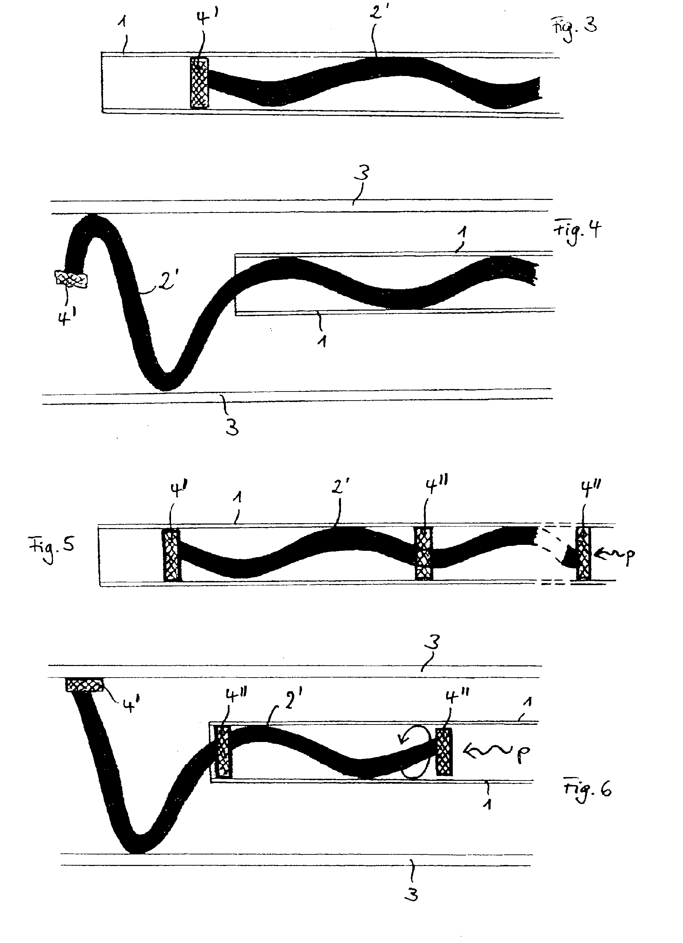

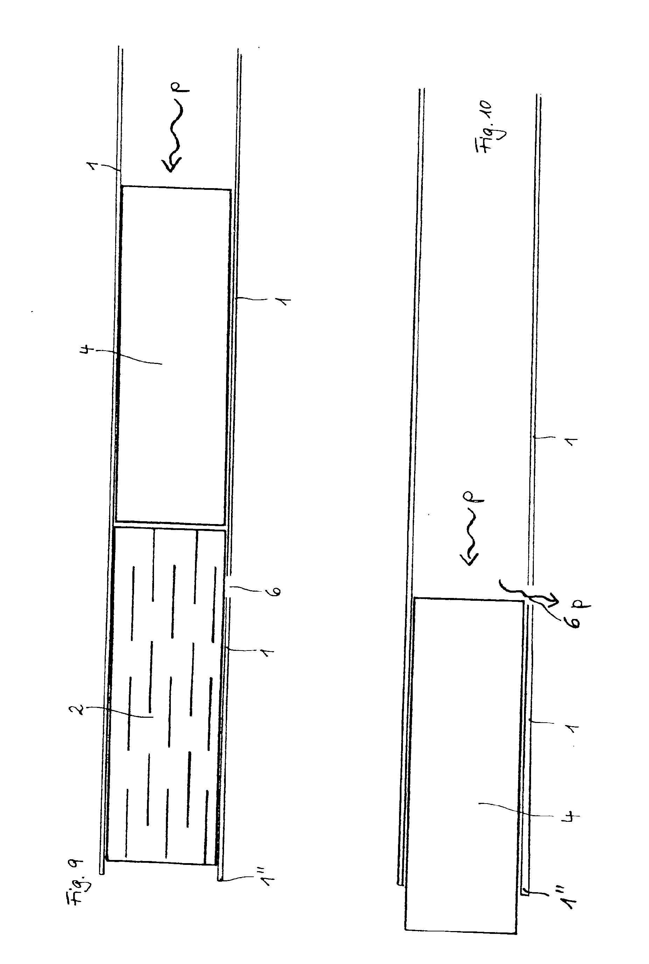

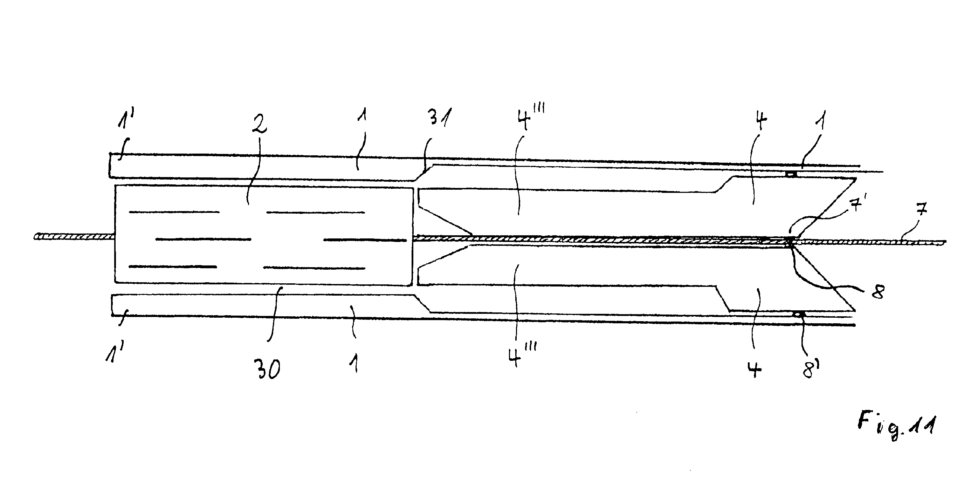

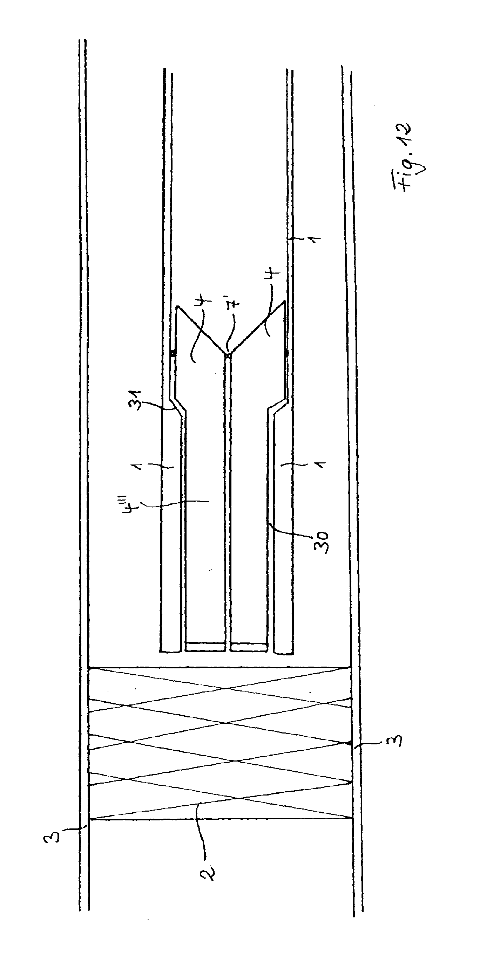

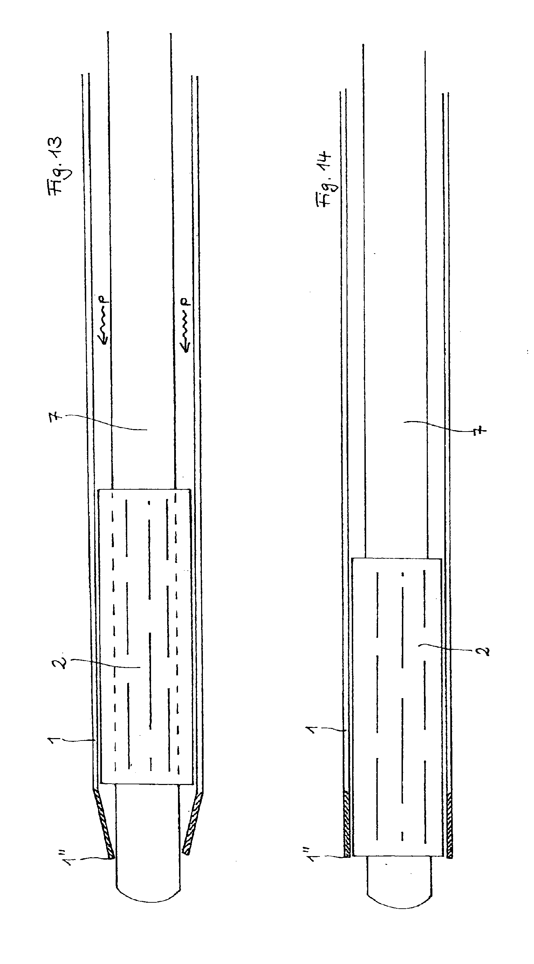

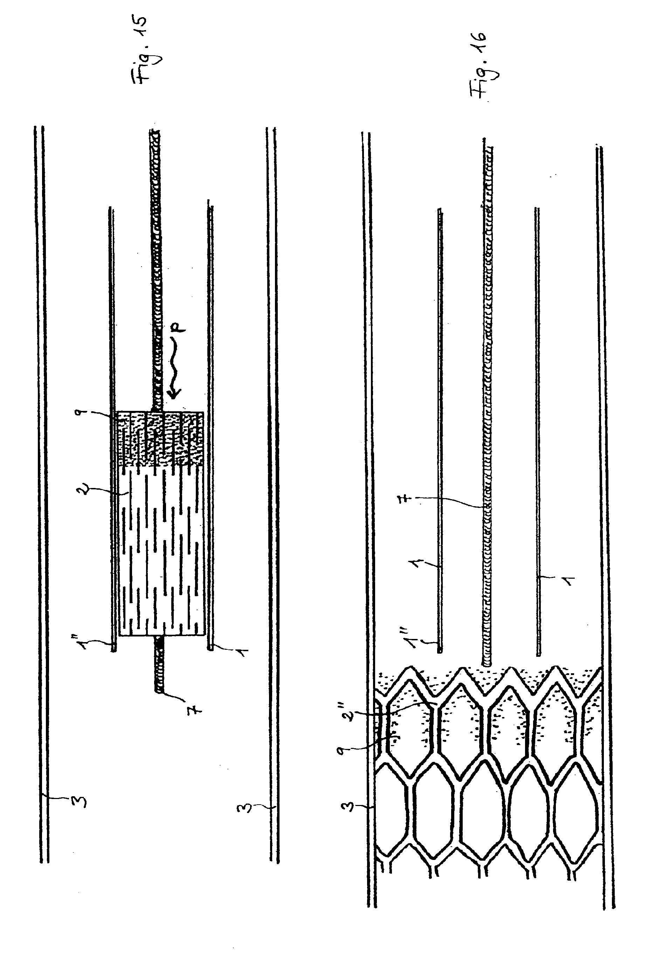

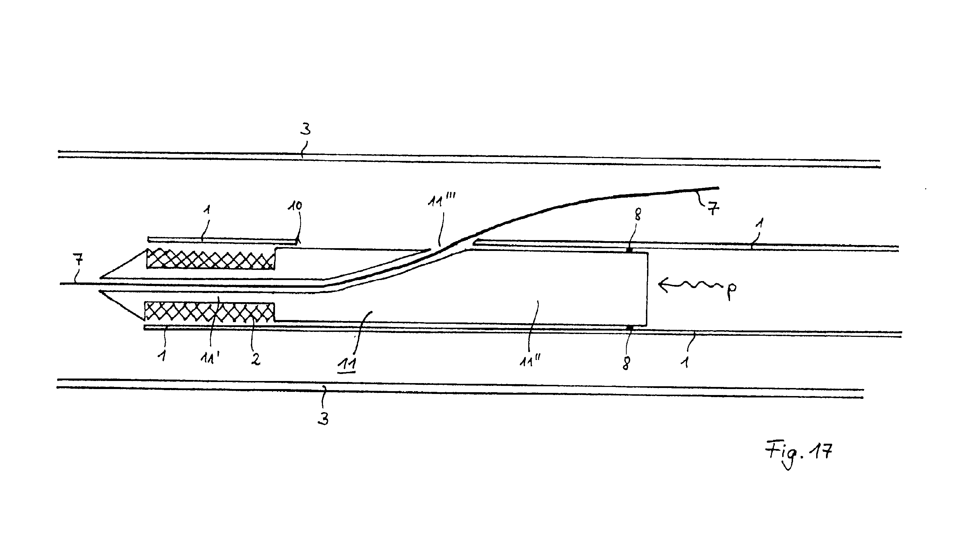

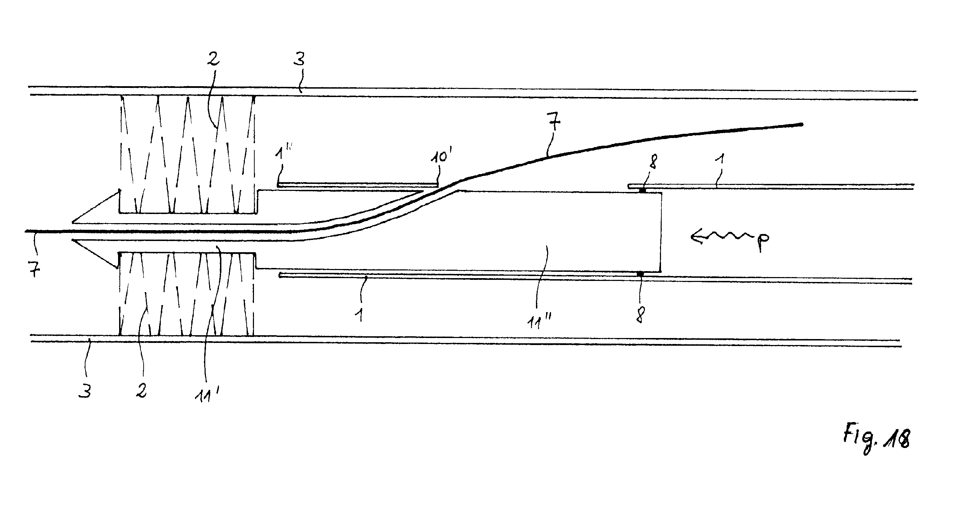

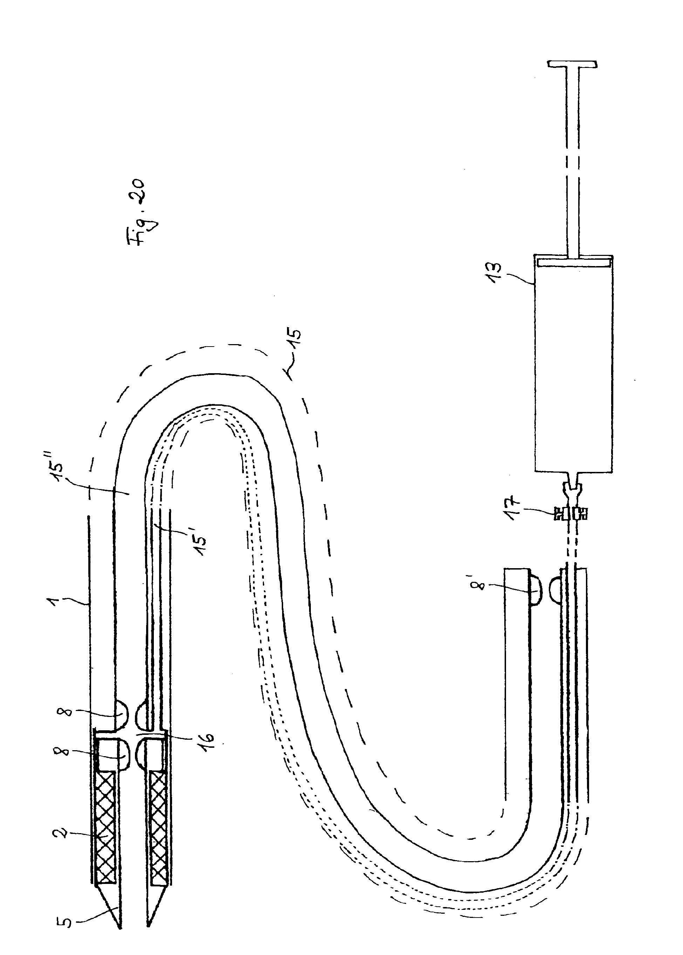

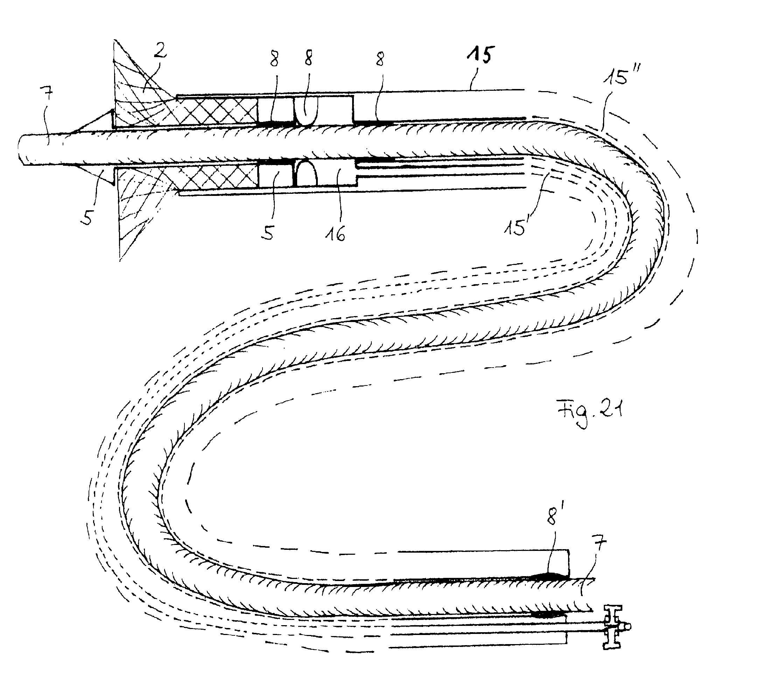

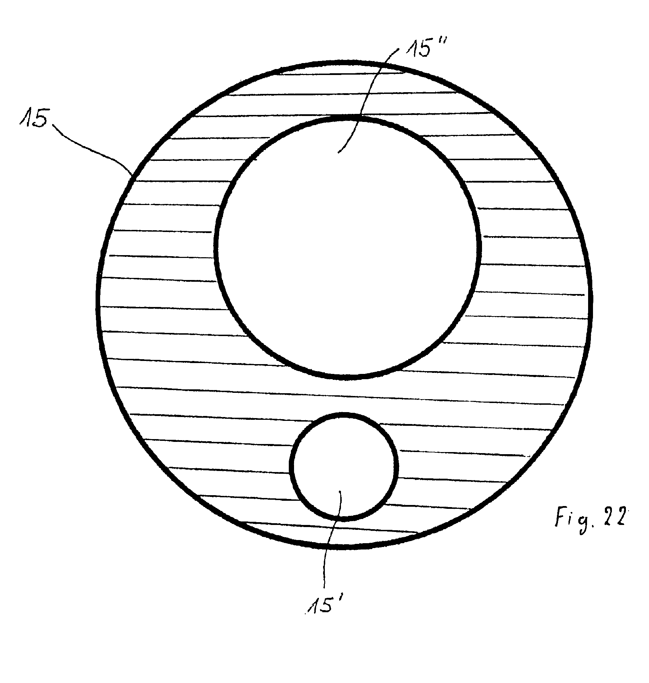

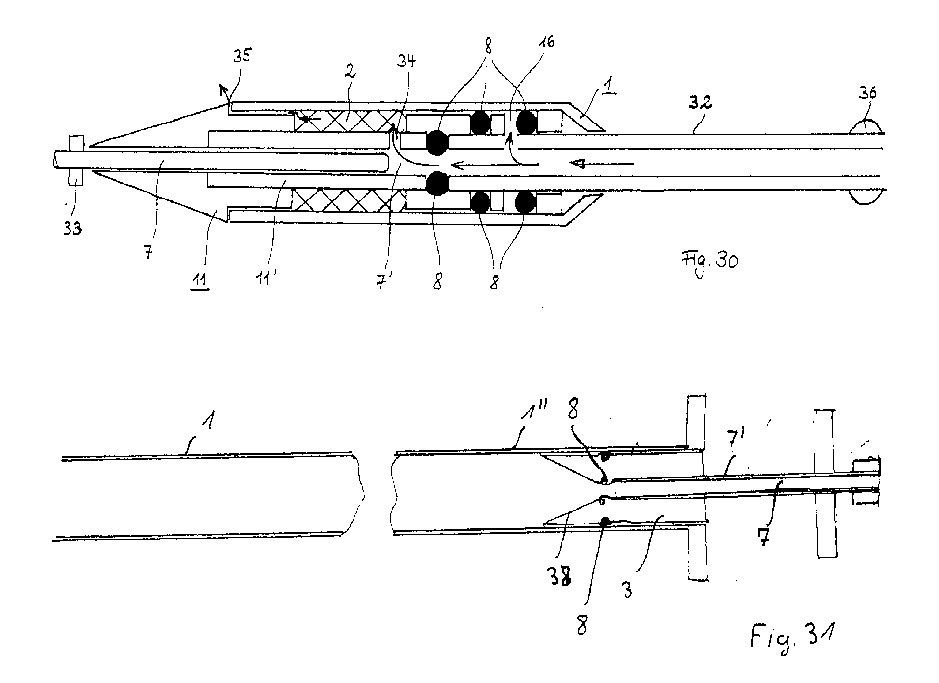

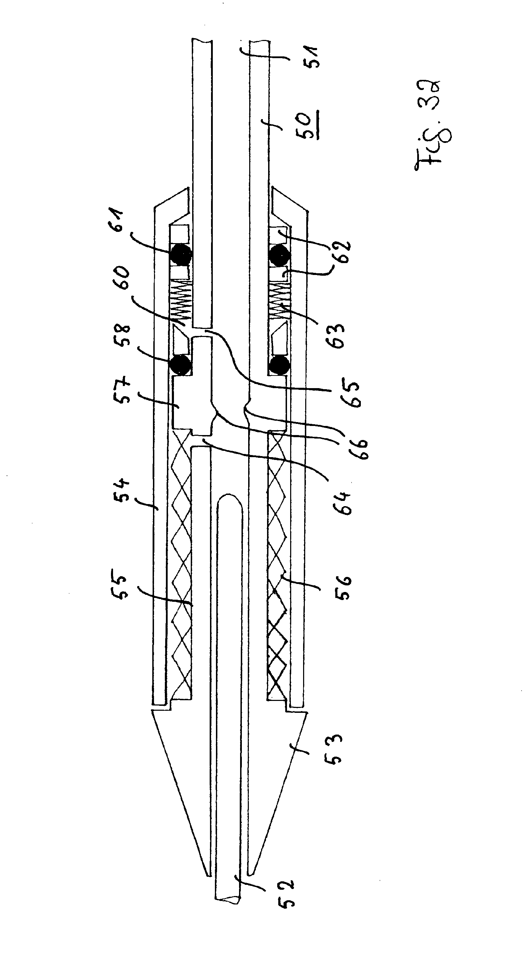

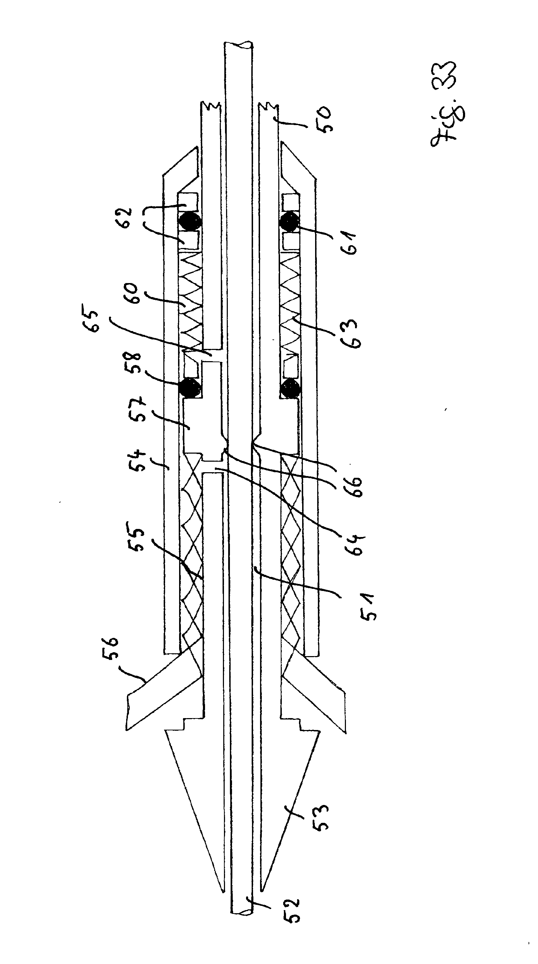

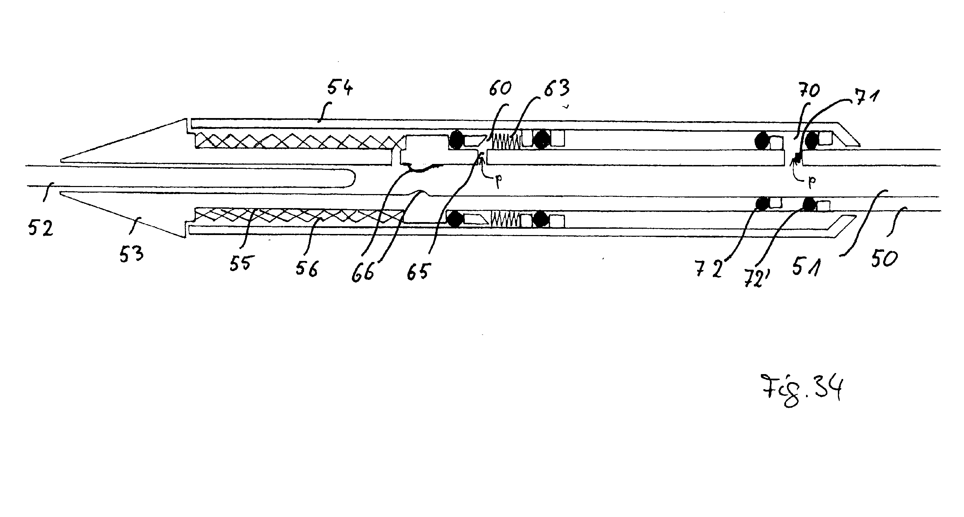

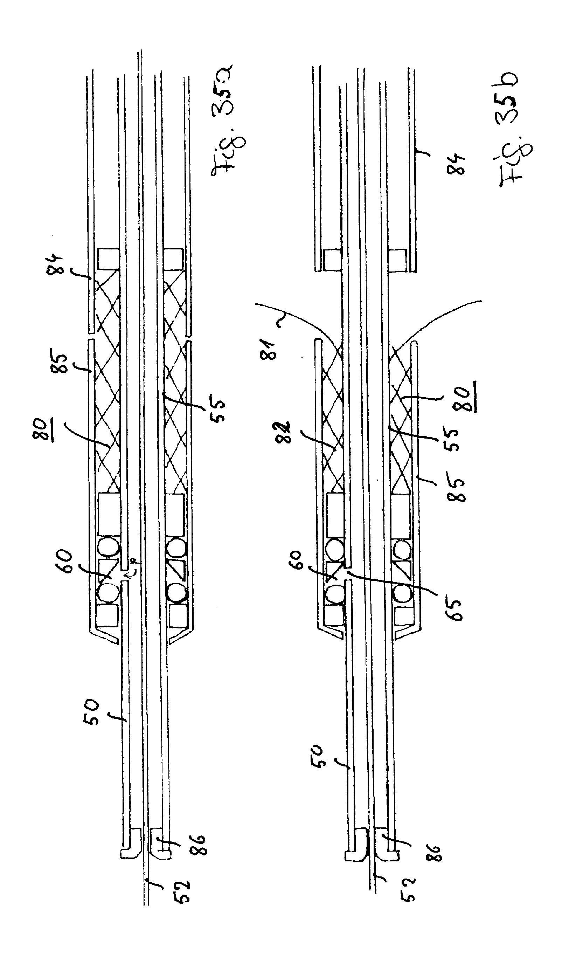

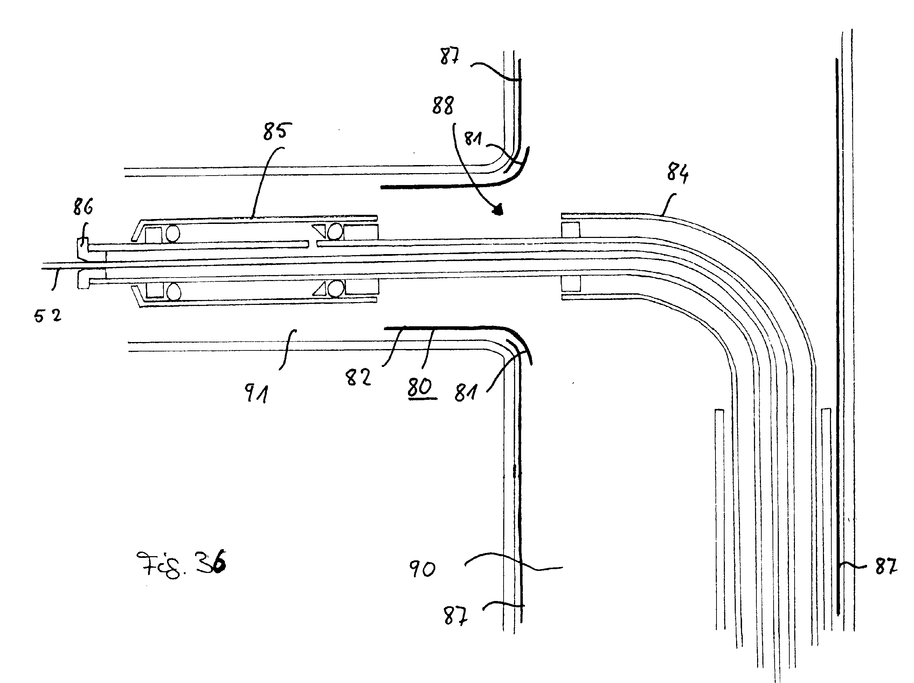

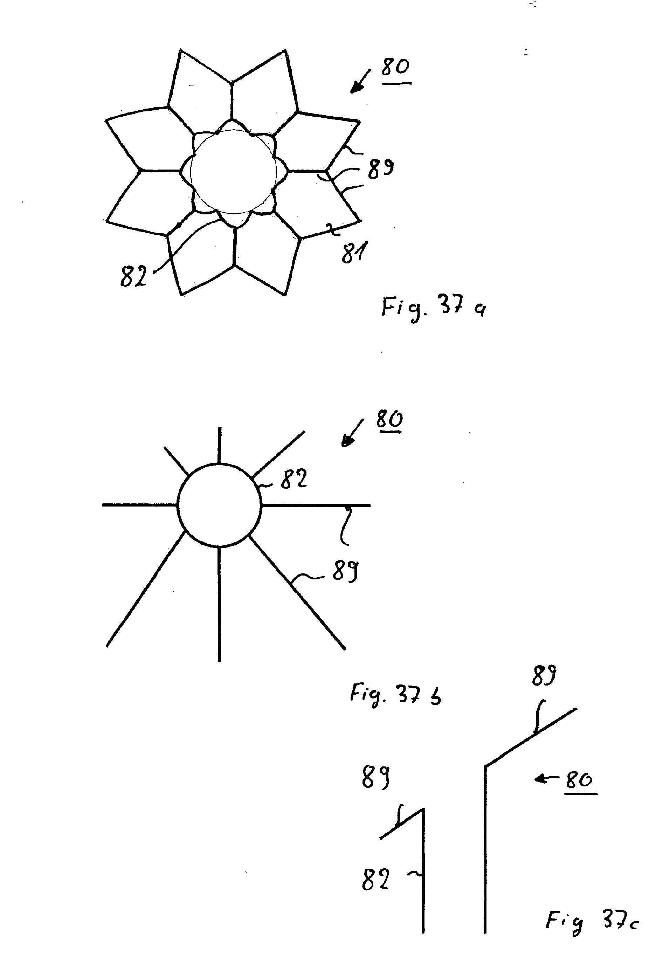

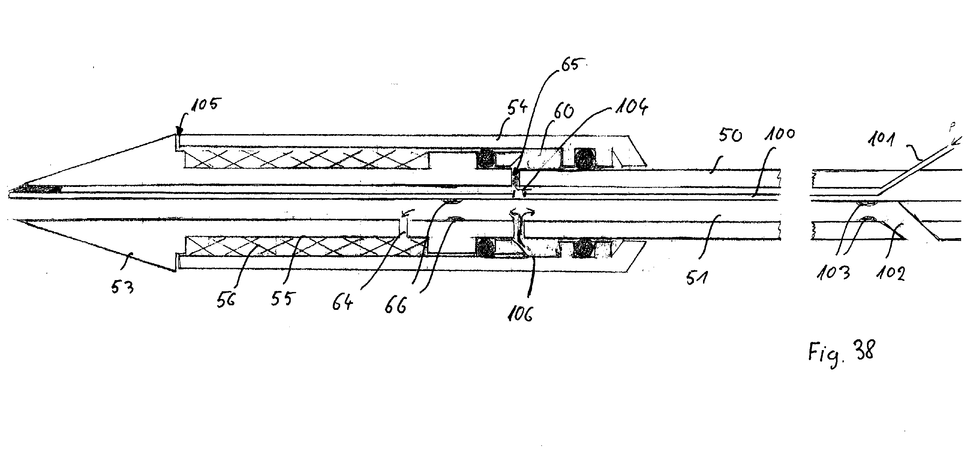

   Fig. 4 zeigt den Spiralstent 2' mit distalem Kolben 4' im teilweise implantierten Zustand. Der Kolben 4' ist jetzt an Größe kleiner geworden, da er nach Erreichen des Körperhohlorgans 3 durch die Körpersäfte oder durch eine über den Katheter 1 eingebrachte Flüssigkeit abgebaut wird und an Größe abnimmt, also schmilzt.
Figur 5:
   Fig. 5 zeigt einen im Katheter 1 längs gestreckten Spiralstent 2' mit einem distalen Kolben 4 und mehreren zusätzlichen kolbenförmigen Erweiterungen 4''. Der an den Spiralstent 2' distalseitig angeformte Kolben 4' kann einen größeren Durchmesser aufweisen als die nachgeschalteten kolbenförmigen Erweiterung 4'', ist aber jedenfalls so groß, dass er das Lumen des Katheters 1 ausfüllt.
Figur 6:
   Das Druckmittel, das von proximalseitig in den Katheter 1 eingeleitet wird, fließt an dem proximalseitig angeformten kolbenförmigen Erweiterungen 4'' vorbei, wodurch zunächst der distalseitig angeformte Kolben 4' aus dem Katheter 1 herausgetrieben wird. Dann folgen die kolbenförmigen Erweiterungen 4'', die den restlichen Anteil des Spiralstents 2' aus dem Katheter 1 austreiben. Hierdurch wird garantiert, dass das Druckmedium auch auf das proximale Ende des Spiralstents 2' wirkt und dieses heraus befördert.
   In einem Fall, in dem die Abschnitte zwischen den kolbenförmigen Erweiterungen 4'' vollständig mit Flüssigkeit gefüllt sind, ist es nicht notwendig, dass diese kolbenförmigen Erweiterung 4'' eine Lekage erlauben, da Flüssigkeiten bekanntlich inkompressibel sind und somit die den Vorschub vermittelnde Druckfraft auch ohne dies fortgeleitet wird.
   Der distale Kolben 4' ist auf dem Draht/Spiralstent so angebracht, dass er sich an die Gefäßwand des zu behandelnden Körpergefäßes 3 anlegt, um Strömungsturbulenzen möglichst gering zu halten.
Figur 9:
   Fig. 9 zeigt einen Längsschnitt durch die Katheter-Kolben-Stent-Vorrichtung. Ein Kolben 4 ist in dem im Patientenkörper angeordneten Katheter 1 wie in einem Zylinder eingebracht und wird durch einen Druck p von proximalwärts in Richtung der distalen Katheteröffnung 1" im Patientenkörper vorgeschoben. Vor dem Kolben befindet sich der Stent 2 in seinem noch nicht entfalteten Zustand (low profile). Kolben 4 und Stent 2 samt Katheter 1 sind flexibel, so dass die Vorrichtung auch in geschlängelt verlaufenden Arterien benutzt werden kann.
   Die Wand des Katheter 1 weist ein Seitloch 6 auf, aus dem das Druckmedium aus dem Katheter 1 hinaus in das Körperhohlorgan oder -gefäß 3 des Patienten entweichen kann, um den Vortrieb des Kolbens 4 abzubremsen. Dies soll verhindern, dass nach Herausschieben des Stents 2 der Kolben 4 aus dem Katheter 1 in das zu behandelnde Körperhohlorgan oder -gefäß 3 des Patienten befördert wird.
Figur 10:
   Das Druckmedium befördert den Stent 2 solange im Katheter 1 nach distalwärts, bis der Kolben 4 das Seitloch 6 der Katheterwandung passiert hat. Anschließend kann das Druckmedium durch das freigegebene Seitloch 6 entweichen, wodurch ein weiterer Vorschub des Kolbens 4 unterbleibt und der Kolben 4 nicht den Katheter 1 verlassen kann.
Figur 11:
   Der Stent 2 ist am distalen Ende des im Patientenkörper befindlichen Katheters 1 hinter der distalen Katheteröffnung 1' in seiner reduzierten, mithin nicht expandierten Form geladen.
   Der Katheter 1 weist in diesem Bereich ein reduziertes Lumen zur Ausbildung einer Stentkammer 30 auf. Am proximalen Ende der Stentkammer 30 ist eine umlaufende Kante 31 angeformt, die verhindert, dass der Kolben 4, nachdem der Stent 2 aus der Stentkammer 30 befördert worden ist, aus dem Katheter 1 herausrutschen kann. Der Stent 2 wird in diesem Falle nicht direkt vom Kolben 4 herausbefördert, sondern durch ein, dem eigentlichen Kolben 4 vorgeschaltetes, distales Schieberteil 4"', das einen dem Innendurchmesser der Stentkammer 30 entsprechenden Außendurchmesser aufweist und somit in seinem Durchmesser kleiner als der Durchmesser des proximalen Schieberteils des Kolbens 4 ist.
   Konzentrisch im Kolben 4 und Schieberteil 4'" ist ein zentraler Kanal 7' zur Aufnahme eines Führungsdrahtes 7 angeordnet. Der Kanal 7' ist mit einem Dichtungsring 8 versehen, der den Führungsdraht 7 gegenüber den Kolben 4 abdichtet. Ein weiterer Dichtungsring 8' findet sich auch am Außenumfang des Kolbens 4, um eine Leckage des Druckmediums zwischen Kolben 4 und Innenfläche des Katheters 1 zu vermeiden. Das proximale Ende des Kolbens 4 und das distale Ende des Schieberteils 4''' sind trichterförmig ausgehöhlt, um jeweils die Sondierung mit dem Führungsdraht 7 zu erleichtern.
Figur 12:
   Der Stent 2 ist hier durch den Kolben 4 herausgedrückt worden. Das distale Schieberteil 4"' des Kolbens 4 befindet sich jetzt am Anschlag der umlaufenden Kante 31 der Stentkammer 30, wobei ein weiterer Vorschub des Kolbens 4 dadurch verhindert ist, dass der proximale Anteil des Kolbens 4 mit seiner Schulter, die einen größeren Durchmesser aufweist als die umlaufende Kante 31, nicht in den vorderen, in seinem Durchmesser reduzierten Anteil des Katheters 1, mithin in die Stentkammer 30, eintreten kann.
Figur 13:
   Ein in kleinem Durchmesser befindlicher, also nicht expandierter Stent 2 ist im Katheter 1 geladen. Der Innendurchmesser (Lumen) dieses Stents 1 ist dem Außendurchmesser des Führungsdrahtes 7 angepasst. Die distale Öffnung 1" des Katheters 1 ist konisch verengt und übergreift distalseitig die Stufenbildung zwischen Führungsdraht 7 und Stent 2. Der Katheter list im Bereich seiner distalen Öffnung 1' elastisch beweglich. Der Stent2 wird vom Druck p des Druckmediums innerhalb des Katheters 1 nach distalwärts befördert.
Figur 14:
   Fig. 14 zeigt das distalseitige Heraustreten des Stents (2) aus dem Katheter 1. Hierbei erweitert sich die aus einem elastischem Material bestehende Katheteröffnung 1".
Figur 15:
   Fig. 15 zeigt einen Längsschnitt durch die Katheter-Kolben-Stent-Vorrichtung.
   Ein selbst expandierender Stent 2 ist in seinem kleinen Durchmesser im Katheter 1 geladen. Das proximale Ende eines solchen Stents 2 dient als Kolben, wobei über eine gewisse Distanz die Zwischenräume zwischen den Stentstreben und der Zwischenraum zwischen Stent und Führungsdraht 7 durch eine sich im Blut schnell auflösende Substanz, z.B. ein Polymer 9, abgedichtet sind. Während des Austritts des Stents 2 aus der distalen Katheteröffnung 1" wird das Polymer 9 vom Druckmedium, z.B. Gas oder einer bestimmten Flüssigkeit, aufgelöst, wodurch der Stent 2 sich bestimmungsgemäß entfaltet.
   In einer anderen Ausführung ist es auch möglich, dass das Druckmedium, z.B. ein Gas oder bestimmte Flüssigkeiten, dieses Polymer 9 nicht auflösen. Das Polymer wird in diesem Falle erst nach Austritt des Stents 2 in das Gefäßsystem durch die Körperflüssigkeiten, z.B. Blut oder Darmsäfte, aufgelöst, wobei dann der Stent 2 seine Gebrauchsgröße erreicht.
Figur 16:
   Figur 16 zeigt einen selbstexpandierenden, bereits implantierter Stent 2 nach Fig. 15, an dessen Stentstreben 2" noch Reste des Polymers 9 haften.
Figur 17:
   Figur 17 zeigt einen Längsschnitt durch eine Katheter-Kolben-Stent-Vorrichtung nach dem Rapid-Exchange-System (schnell einführbares System, auch Monorail genannt).
   Der Angiographie-Führungsdraht 7 verläuft hier nicht zentral durch den gesamten Katheter 1, sondern nur im distalen Anteil des Katheters 1. Der Führungsdraht 7 wird an der Spitze eines Trägers 11, welcher ein Stent-Bett 11' sowie ein nachgeschaltetes Kolbenteil 11" beinhaltet, zunächst zentral eingeführt und dann seitlich aus dem Kolbenteil 11'' durch ein Träger-Seitloch 11''' ausgeführt, und verläuft dann neben dem Katheter 1 über die Kathetereinführschleuse nach außen.
   Gegenüber dem für den Führungsdraht 7 vorgesehenen Träger-Seitloch 11''' ist ein Längsschlitz 10 im Katheter 1 vorgesehen, der ein Vorwärtsgleiten des Kolbenteils 11'' im Katheter 1 erlaubt, während der Führungsdraht 7 seitlich heraustritt. Das Kolbenteil 11'' ist dabei gegenüber dem Katheterlumen durch einen Dichtungsring 8 abgedichtet. Das den Stent 2 transportierende Kolbenteil 11'' wird durch den Druck p des Druckmediums vorgeschoben.
Figur 18:
   Figur 18 zeigt den einen Stent 2 gemäß Figur 17 nach dessen Freisetzung aus dem Katheter 1.
   Das Kolbenteil 11 wurde durch den Druck p vollständig nach distalwärts in Richtung der distalen Katheteröffnung 1' vorgeschoben bis das nun im Körperhohlorgan 3 freiliegende Stent-Bett 11' den Stent 2 zur Entfaltung entläßt. Ein weiteres Austreten des Kolbenteils 11'' wird dadurch verhindert, dass der Führungsdraht 7 an der distalen Kante 10' des Längsschlitzes 10 im Katheter 1 sperrt. In diesem Zustand ist der Stentträger 11 so weit aus dem Katheter 1 herausgefahren, dass das Stent-Bett 11 sich außerhalb des Katheters 1 im Körperhohlorgan 3 befindet und der Stent 2 sich hier auf seine Bestimmungsgröße ausdehnen kann. Der hintere Teil des Trägers 11, der als Kolbenteil 11'' wirkt, verbleibt jedoch im Katheter 1. Das Katheter-Kolben-System kann nach Freisetzung des Stents 2 in seiner Gesamtheit über den Führungsdraht 7 zurückgezogen werden, wobei der Führungsdraht 7 auch im entfalteten Stent 2 belassen werden kann, um eventuell weitere therapeutische Maßnahmen zu ergreifen.
Figur 20:
   Figur 20 zeigt einen doppellumigen Katheter 15, mit einem engeren Lumen 15' und einem weiteren Lumen 15'', in einer Stellung bevor der Führungsdraht 7 in den Katheter 1 eingeführt worden ist. Über das engere Lumen 15' wird die Vorrichtung entlüftet, indem das Druckmedium mittels einer Pumpe 13 durch das enge Lumen 15' in eine Hauptdruckkammer 16 geleitet wird und dabei über die noch offenen Dichtungsringe 8 entweichen kann. Nach Austritt der Spülflüssigkeit, die meist gleichbedeutend mit dem Druckmedium ist, wird ein Verschlusshahn 17 verschlossen und der Führungsdraht 7 über die distale Spitze des Trägers 5 eingebracht.
Figur 21:
   Fig. 21 zeigt Vorgang der Stentfreisetzung mit der in Figur 20 beschriebenen Vorrichtung, nach Einführung des Führungsdrahtes 7 in das weitere Lumen 15 " . Das System wird dann an den Zielort verbracht und über das engere Lumen 15' des Katheters 1 die Druckbeaufschlagung zur Stentfreisetzung durchgeführt. In der Darstellung gemäß Fig. 21 ist der Stent 2 bereits teilweise entfaltet. Der Träger 5 hat an seinem distalen Teil zwei Dichtungen 8. Die erste Dichtung dichtet dabei einen Zwischenraum zwischen dem Führungsdraht 7 und dem zentralen Kanal im Träger 5 ab. Die zweite Dichtung 8 dichtet hingegen den äußeren Zwischenraum zwischen Träger 5 und dem doppellumigen Katheter 15 ab. Das hämostatische Ventil 8' am distalen Katheterende dichtet das größere Lumen 15'' bei eingeführtem Führungsdraht 7 ab.
Figur 22:
   Fig. 22 zeigt den Querschnitt durch den doppellumigen Katheter 15, welcher in Fig. 20 und 21 im Längsschnitt gezeigt ist. Das engere Lumen 15' für die Druckbeaufschlagung ist, verglichen mit dem weiteren Lumen 15'' für den Führungsdraht 7 vergleichsweise dickwandig, also von einer großen Menge Wandmasse umgeben.
Figur 30:
   Figur 30 zeigt abermals eine alternative Ausführung der Katheter-Stent-Vorrichtung im Längsschnitt. Gemäß der hier gewählten Darstellung wird der noch nicht expandierte Stent 2 in einem Hüllkatheter 1 mittels eines Strentträgers 11 durch den Katheter 1 bewegt. Dabei weist der Stenträger 11 einen zentralen Kanal 7'zur Aufnahme des Führungsdrahts 7 auf. Der Führungsdraht 7 wird in dieser Ausführung distalseitig zunächst bis zu einem von einer den Führungsdraht 7 konzentrisch umschließenden Muffe 33 in den zentralen Kanal 7 eingeführt. In dieser Stellung ragt die proximale Spitze des Führungsdrahts 7 bis in den vom Stent 2 umschlossenen Bereich des Stent-Betts 11'. In dieser Stellung kann nun die Vorrichtung mittels einer Spülflüssigkeit, respektive dem Druckmedium gespült werden. Aufgrund der entsprechenden Abdichtung durch die Dichtungen 8 wird dabei eine in diesem Bereich zwischen Führungsdraht 7, Stentträger 11 und Hüllkatheter 1 ausgebildete Duckkammer 16 ebenso gespült wie der Stent 2 als solcher, wobei hierbei die Spülflüssigkeit gemäß der Pfeildarstellung in der Zeichnung proximalseitig in den zentralen Kanal 7' eingebracht wird und durch eine Spülöffnung 34 im Bereich des Stent-Betts 11' über den Stent 2 und schließlich einen Spülspalt 35 zwischen Stent 2 und der distalen Spitze des Stentträgers 11 ebenso ausströmen kann wie etwa noch im Stent 2 oder in der Vorrichtung insgesamt verbliebene Luft. Die Spülung bewirkt somit die vollständige Evakuierung der gesamten Vorrichtung.
   Wenn anschließend der Führungsdraht 7 in proximaler Richtung weiter vorgeschoben wird, verhindern anschließend die den Führungsdraht 7 dann konzentrisch umschließenden Dichtungen 8 ein über den Bereich dieser Dichtungen 8 hinaus weiteres Vordringen der Spülflüssigkeit in distaler Richtung und damit auch des Druckmediums in distaler Richtung. Statt dessen wird die nunmehr abgeschlossene Druckkammmer 16 geflutet und druckbeaufschlagt, so dass infolgedessen der Hüllkatheter 1 in proximaler Richtung zurückgleitet bis durch dieses Zurückgleiten die Druckkammer 16 geöffnet wird und damit die Rückwärts-Bewegung des Hüllkatheters 1 gestoppt ist. In dieser Stellung ist aber der Stent 2 bereits bestimmungsgemäß freigesetzt. Zusätzlich kann eine proximale Anschlagmuffe 36 auf dem Innenkatheter 32 die Rückwärtsbewegung des Hüllkatheters 1 in einer definierten Position stoppen.
Figur 31:
   Figur 31 zeigt eine mögliche Ausführung des proximalen Katheterendes des Katheters 1 im Längsschnitt. Dabei ist in Proximalteil 1" des Katheters 1 ein Druckkolben 36 eingeführt. Der Druckkolben 37 weist ebenfalls einen zentralen Kanal 7' für den Führungsdraht 7 auf, der proimalseitig in einer trichterförmigen Erweiterung 38 zur erleichterten Einführung des Führungsdrahtes 7 mündet. Dabei ist der in das proximale Katheterende eingeführte Druckkolben 37 mittels entsprechender Dichtungen 8 sowohl im Bereich den zentralen Kanals 7 sowie gegenüber dem Katheter 1 strömungsdicht und damit auch druckdicht abgedichtet. Bei vollständig geflutetem System bewirkt demnach das weitere Einführen des Druckkolbens 37 aufgrund der im System als Druckmedium befindlichen inkompressiblen Flüssigkeit je nach Gestaltung der distalen Anordnung der Katheter-Stent-Vorrichtung ein Austreiben des Stents aus dem Katheter 1 oder ein Zurückgleiten das Katheters oder eines zusätzlichen Hüllkatheters. In allen Fällen wird der selbstexpandierende Stent 2 bestimmungsgemäß am Implantationsort freigesetzt.
   Figur 32 zeigt eine Katheter-Stent-Vorrichtung mit einem einteiligen Hüllkatheter vor der Implantation im Querschnitt:
   Gemäß der Darstellung in Figur 32 umfasst eine weitere Katheter-Stent-Vorrichtung gemäß dieser Darstellung einen Hauptkatheter 50 mit einem konzentrisch angeordneten Zentralkanal 51. Dieser Hauptkatheter 50 kann durch ein hämostatisches Ventil einer Katheterschleuse in den Körper des Patienten implantiert werden kann. Dabei wird der Hauptkatheter 50 bestimmungsgemäß über einen Führungsdraht 52 geschoben. Der Vorteil besteht darin, dass das System verminderte Reibungskräfte überwinden muss, so dass ein innerer Schiebekatheter innerhalb des Hauptkatheters 50 bei dieser Lösung nicht notwendig ist. Die Katheter-Stent-Vorrichtung kann daher insgesamt länger ausgebildet sein und ist überdies auch flexibler als konventionelle Stent-Platzierungssysteme, wie noch deutlich werden wird. Der Hauptkatheter 50 ist im Bereich hinter seiner distalseitigen Spitze 53 von einem Hüllkatheter 54 konzentrisch umschlossen.

Der Hüllkatheter 54 umschließt seinerseits ein an den Hauptkatheter 50 angeformtes Stent-Bett 55, wobei im Bereich des Stent-Betts 55 der zu implantierende Stent 56 in dieser Darstellung noch im komprimierten Zustand aufgenommen ist. Das Stent-Bett 55 ist distalseitig durch einen in Richtung des Hüllabschnitts vorspringenden Außenring des Hauptkatheters 50 abgeschlossen, an dem sich in proximaler Richtung eine Ringdichtung anschließt, die die in proximaler Richtung sich anschließende Druckkammer 60 gegenüber dem Bereich des Stent-Betts 55 abschließt. Die Druckkammer 60 ist ihrerseits proximalseitig durch eine weitere Ringdichtung 61 und einen Ringkolben 62 abgeschlossen. In der Druckkammer 60 selbst ist wenigstens eine den Hauptkatheter 50 konzentrisch umschließende Druckfeder 63 angeordnet, deren Druckkraft unmittelbar auf den Ringkolben 62 einwirkt. Dabei ist das Stent-Bett 55 und die Druckkammer 60 jeweils von dem Hüllkatheter 54 umschlossen. Im Bereich des Stent-Betts 55, wie auch im Bereich der Druckkammer 60 weist jeweils die Wandung des Hüllkatheters 54 eine Stent-Bett-Spülöffnung 64 sowie eine Druckkammerspülöffnung 65 auf. Die beiden Spülöffnungen 64 und 65 sind jeweils mit dem Zentralkanal 51 strömungstechnisch verbunden. Dabei ist zwischen der Stent-Bett Spülöffnung 64 und der Druckkammerspülöffnung 65 an die Innenwandung des Hauptkatheters 50 ein innerer Ringwulst 66 angeformt, der bei bestimmungsgemäßer Dimensionierung des Führungsdrahts 52 und Positionierung strömungsdicht mit dem Führungsdrahts 52 abschließt.

Aus der in Figur 32 gezeigten Darstellung ist ersichtlich, dass es vor der Implantation leicht möglich ist, die Katheter-Stent-Vorrichtung distalseitig etwa mit einem Finger zu verschließen, um dann von der proximalen Seite die Vorrichtung zu spülen, wobei in diesem Falle die Spülflüssigkeit, vorzugsweise eine körperverträgliche Kochsalzlösung, durch die Spülöffnungen 64 und 65 sowohl das Stent-Bett 55, als auch die Druckkammer 60 spült und etwa eingeschlossene Luft evakuiert.

In einem weiteren Schritt wird dann der Hauptkatheter 50 bestimmungsgemäß über den Führungsdraht 52 implantiert und gelangt auf diesem Wege an den bestimmungsgemäßen Implantationsort.

Figur 33 zeigt die in Figur 32 gezeigte Katheter-Stent-Vorrichtung während der Implantation:

Ebendort wird dann gemäß der Darstellung in Figur 33 durch Druckbeaufschlagung der Druckkammer 60 des Hüllkatheters 54 in proximaler Richtung ausgetrieben. Hierzu wird das Druckmedium, vorzugsweise wiederum eine körperverträgliche Kochsalzlösung, proximalseitig durch den Zentralkanal 51 eingespült. In der Stellung gemäß Figur 33 ist nun aber über den inneren Ringwulst 66 die Stent-Bett-Spülöffnung 64 gegenüber der proximalseitig einströmenden Flüssigkeit strömungstechnisch abgeriegelt, so dass der Flüssigkeitsdruck in die Druckkammer 60 eingeleitet wird. Die Druckbeaufschlagung der Druckkammer 60 führt dann dazu, dass in Verbindung mit der durch die Druckfeder 63 eingeleiteten Kraft, die auf dem proximalseitig angeordneten Ringkolben 62 einwirkt, dass die Druckkammer 60 expandiert und den Hüllkatheter 54 in proximaler Richtung zurückgeschoben wird und hierdurch der komprimierte Stent 56 nach und nach freigegeben wird. Der Stent 56 weitet sich dann bestimmungsgemäß von einem reduzierten Implantationslumen auf das bestimmungsgemäße Lumen in dem zu behandelnden Körpergefäß auf. Die Spülflüssigkeit zur Druckbeaufschlagung der Druckkammer 60 wird üblicherweise durch ein hier nicht weiter dargestelltes Seitloch in den Hauptkatheter 50 gespült.

Die Dichtwirkung des inneren Ringwulstes 66 kann dabei durch Wärmeeinwirkung, also durch thermoplastisch Umformung, unterstützt werden.

Durch die Druckbeaufschlagung der Druckkammer 60 muss zunächst die Haftungsreibung des Hüllkatheters 54 relativ zum Hauptkatheter 50 und zu dem eingeschlossenen Stent 56 überwunden werden. Die Überwindung der im Vergleich zur Gleitreibung größeren Haftungsreibungskraft kann oftmals nicht alleine durch den hydraulischen Druck der Druckkammer 60 überwunden werden, so dass insoweit die in der Druckkammer 60 zusätzlich angeordnete Druckfeder 63 zur Reduktion der durch Druckbeaufschlagung aufzubringenden Kraft erforderlich ist. Allerdings ist bei der Dimensionierung der Druckfeder 63 darauf zu achten, dass die Druckfeder 63 so dimensioniert ist, dass sie nicht schon für sich alleine die anstehende Haftungsreibung überwinden kann.

Als Druckfeder 63 kommen dabei eine Tellerfeder und/oder eine Scheibenfeder in Frage. Die Druckfester 63 steht also stellvertretend für eine Druckfederanordnung in der Druckkammer, die im Rahmen der Erfindung bedarfsweise auch aus mehreren ggf. sogar unterschiedlichen Federn bestehen kann.

Sobald der Stent 56 vollständig expandiert ist, kann dann der Hauptkatheter 50 mitsamt dem Hüllkatheter 54 über den Führungsdraht 52 zurückgezogen werden, wobei der expandierte Stent 56 in seiner bestimmungsgemäßen Stellung in dem zu behandelnden Körpergefäß zurückbleibt.

Figur 34 zeigt eine alternative Ausgestaltung der Katheter-Stent-Vorrichtung mit zwei Druckkammern innerhalb des Hüllkatheters:

Eine alternative Ausgestaltung der Katheter-Stent-Vorrichtung ist in Figur 34 dargestellt. Gemäß der Darstellung in Figur 34 umschließt hier der Hüllkatheter 54 neben der ersten Druckkammer 60 eine zweite Druckkammer 70. Durch die zwei hintereinander geschalteten Druckkammern 60, 70 wird die auf den Hüllkatheter 54 einwirkende Kolbenfläche vergrößert, so dass der über das Druckmedium und die Druckbeaufschlagung der Druckkammern 60 und 70 aufzuwendende Druck zum Verschieben der Hüllkatheter 54 geringer gewählt werden kann.

Als Dimensionierungsvorschrift kann gelten, dass der von den beiden Druckkammern 60 und 70 überstrichene Raum in Längsrichtung mindestens größer, gleich der Länge des Stent-Betts 55 sein sollte. Dabei ist die erste Druckkammer 60 analog aufgebaut, wie bei der vorstehend erläuterten Ausführung. Die zweite Druckkammer 70 weist eine weitere Druckkammerspülöffnung 71 sowie weitere Ringdichtungen 72, 72' auf. Die beiden Dichtungen 72, 72' sind jeweils vor und hinter der weiteren Spülöffnung 71 der weiteren Druckkammer 70 angeordnet.

Die Variante mit zwei Druckkammern 60, 70 zur Reduktion des mittels des Druckmediums aufzubauenden Drucks empfiehlt sich insbesondere bei größeren Stentlängen.

Figur 35 a zeigt eine Katheter-Stent-Vorrichtung mit einer zweigeteilten Katheterhülle Implantation im Querschnitt:

Figur 35 b zeigt die in Figur 35 a gezeigte Katheter-Stent-Vorrichtung Implantation im Querschnitt.

Die Darstellungen gemäß den Figuren 35 a und b betrifft einen anderen, zweiteiligen, aber einstückigen Stent 80. Der fragliche Stent 80 eignet sich insbesondere für Stenosen im Bereich der Öffnung von abgehenden Arterien-Ästen aus einem Hauptgefäß, z. B. bei einer ostialen Nierenarterienstenose. Es ist besonders schwierig, diese Stenosen mit einem Stent zu versorgen, da bei der Implantation der Stent meist entweder zu weit distal vorgeschoben, also in die abgehende Nierenarterie implantiert wird, und im Ergebnis die zumeist im Bereich der Verzweigung angeordnete Stenose dann durch den Stent nicht mehr abgedeckt wird, mithin das Blutgefäß in diesem Bereich nicht hinreichend eröffnet ist oder umgekehrt der Stent zu weit proximalseitig in das Lumen der Aorta übersteht und hierdurch der Blutfluss in diesem Gefäß behindert ist.

Für derartige Stenosen im Verzweigungsbereich von Körpergefäßen wird daher in Verbindung mit der erfindungsgemäßen Katheter-Stent-Vorrichtung gemäß der Darstellung in den Figuren 35 a und b ein anderer Stent 80 mit einem proximalen Stentabschnitt 81 und einem distalen Stentabschnitt 82 vorgeschlagen. Die beiden Stentabschnitte ergänzen sich zu einem gemeinsamen Stent 80, wobei der distale Stent im Sinne ein im weitesten Sinne herkömmlicher Stentabschnitt eines selbstexpandierenden Stents ist, also ein Stent, der sich von einem geringeren Implantationslumen am Implantationsort in hier nicht weiter interessierender Weise nach Freisetzung auf ein bestimmungsgemäßes, größeres Lumen ausdehnt und somit das Blutgefäß in diesem Bereich eröffnet, insbesondere also die erwähnte Stenose im Verzweigungsbereich der Körpergefäße abdeckt. Der proximale Stentabschnitt 81 eröffnet sich im Implantationsort jedoch zu einem Ringflansch 83. Dies gelingt durch in den Stent 80 in diesem proximalen Stentabschnitt 81 eingearbeitete Stentstreben, die radial nach außen streben und sich nach Freisetzung des fraglichen Stentabschnitts 81 derart radial aufweiten, dass hierdurch der Ringflansch 83 am Implantationsort bestimmungsgemäß eröffnet wird.

Im Falle der Implantation eines derartigen Stents 80 genügt es, wenn der Stent 80 zunächst in dem ungefähren Verzweigungsbereich poitioniert wird und dort bestimmungsgemäß expandiert wird, wobei sich hierbei zunächst bestimmungsgemäß der Ringflansch 83 aufweitet. Im Rahmen der weiteren Implantation kann dann der Stent 80 in distaler Richtung so weit vorgeschoben werden, bis der Ringflansch 83 an der Aortenwand abschließt und ein weiteres Vorschieben des Stents 80 in distaler Richtung verhindert.

Unterstützend können die abgehenden radialen Drähte der Eröffnung des Ringlanschs röntgenmarkiert sein, so dass während der Implantation überprüfbar ist, ob der Ringflansch 83 bereits seine bestimmungsgemäße Lage in unmittelbarem Anschluss an die Aortenwand im Bereich der Verzweigung erreicht hat. Die Katheter-Stent-Vorrichtung ist gemäß den Figuren 35 a und b optimal auf die Implantation eines derartigen Spezialstents ausgerichtet, wie im Rahmen der nachstehenden Erläuterung deutlich werden wird.

Gemäß der Darstellung in Figur 35a ist auch der Hüllkatheter 54 zweigeteilt wobei es sich hier um eine echte Aufteilung in zwei unterschiedliche Hüllkatheter handelt, die voneinander separiert sind. Es handelt sich also um einen proximalen Hüllkatheterabschnitt 84 und einen distalen Hüllkatheterabschnitt 85. Die beiden Hüllkatheterabschnitte 84 und 85 umschließen konzentrisch den Hauptkatheter 50, der distalseitig mit einem Verschluss 86 strömungsverschlossen ist. Der Hauptkatheter 50 enthält wiederum einen Zentralkanal 51 für einen konzentrisch in dem Hauptkatheter 50 aufgenommenen Führungsdraht 52. Der Führungsdraht durchdringt auch den Katheterverschluss 86, so dass der Hauptkatheter 50 über den Führungsdraht 52 bestimmungsgemäß an den Implantationsort, also insbesondere in den Verzweigungsbereich abgehender Arterien-Äste aus einem Hauptgefäß verbracht werden kann.

Eine weitere Besonderheit der erfindungsgemäßen Katheter-Stent-Vorrichtung liegt darin, dass die Druckkammer 60 distalseitig zum Stent-Bett 55 angeordnet ist. Dies ist auch der Grund, warum der Katheterverschluss 86 distalseitig benötigt wird, nämlich um eine Druckbeaufschlagung der distalseitig angeordneten Druckkammer 60 vornehmen zu können.

Sobald also auch diese Katheter-Stent-Vorrichtung zunächst in der vorstehend beschriebenen Weise vor der Implantation gespült wurde, um etwa eingeschlossene Luft zu evakuieren, wird dann der die Katheter-Stent-Vorrichtung über den Führungsdraht 52 bis in den zu behandelnden Verzweigungsbereich vorgeschoben. Dort wird dann der Stent 80 in zwei weiteren Arbeitsschritten am Implantationsort, wie folgt, implantiert.

Gemäß der Darstellung gemäß Figur 35 b wird in einem ersten Schritt zunächst der proximale Hüllkatheterabschnitt 84 nach außen, also in proximaler Richtung, abgezogen. Der proximalseitige Hüllkatheterabschnitt 84 erstreckt sich deshalb nach außen bis zum eingreifenden Arzt. Durch das Abziehen des proximalen Hüllkatheterhüllabschnitts 84 wird der proximale Stentabschnitt 81 freigesetzt und dieser Stentabschnitt 81 mit seinen radial nach außen weisenden Streben zu dem bereits erwähnten Ringflansch 83 erweitert. Der verbleibende distale Hüllkatheterabschnitt 82 wird mit hydraulischer Kraft entfernt, indem nämlich nun durch den Hauptkatheter 50 die distalseitig angeordnete Druckkammer 60 mit einem Druckmedium beaufschlagt wird und hierdurch die Druckkammer 60 derart expandiert wird, dass der distale Hüllkatheterabschnitt 85 in distaler Richtung vorangetrieben wird, so dass nun auch der distale Stentabschnitt 82 freigeben wird und sich auf sein bestimmungsgemäßes Lumen vor Ort erweitert.

Sinnvoller Weise wird jedoch bevor dieser zweite Expansionschritt durchgeführt wird, die Katheter-Stent-Vorrichtung erst lagerichtig positioniert, indem die Katheter-Stent-Vorrichtung über den Führungsdraht 52 in distaler Richtung vorgeschoben wird, bis der bereits eröffnete Ringflansch 83 an der Gefäßwandung des Hauptgefäßes bestimmungsgemäß anliegt. Dies gelingt entweder, indem bei der Implantation der entsprechende Anschlag bemerkt wird oder indem aufgrund der Röntgenmarkierung der entsprechenden Stentstreben im Bereich des proximalen Stentabschnitts das Anliegen an der Hauptgefäßwandung auf einer bildgebenden Einheit erkannt wird. Erst dann erfolgt die Druckbeaufschlagung der Druckkammer 60 in der vorstehend beschriebenen Weise, um hierdurch den distalen Hüllkatheterabschnitt 85 von dem distalen Stentabschnitt 82 zu entfernen

In einem dritten Arbeitsschritt kann dann der Hauptkatheter 50 mitsamt dem distalen Hüllkatheterabschnitt 85 des Hüllkatheters 54 durch den mittlerweile expandierten Stent 80 hindurch aus dem Nebengefäß in das Hauptgefäß zurückgezogen werden.

Figur 36 zeigt die in Figur 35 a und b dargestellte Katheter-Stent-Vorrichtung nach der bestimmungsgemäßen Expansion des Stents 80 am Implantationsort.

In der Stellung in Figur 36 ist der proximale Hüllkatheterabschnitt 84 bereits von außen in das Hauptgefäß 90 zurückgezogen werden. Der Stent 80 hat sich bereits am Implantationsort, also im Bereich der Verzweigung zwischen dem Hauptgefäß 90 und dem abzweigenden Nebengefäß 91, bestimmungsgemäß aufgeweitet, wobei der Stenttrichter 83 weitgehend an der Wandung des Hauptgefäßes 90 anliegt, während der distale Stentabschnitt 82 sich mit dem erweiterten Lumen, vorzugsweise an der Gefäßwandung des Nebengefäßes 91 in diesem Verzweigungsbereich angelegt hat. Der distalseitige Hüllkatheterabschnitt 85 kann nun in einem letzten Arbeitsschritt über den Führungsdraht 52 durch den erweiterten Stent 80 hindurch zunächst aus dem Nebengefäß 91 und dann aus dem Hauptgefäß 90 bestimmungsgemäß herausgezogen werden.

Eine ebenfalls in Figur 36 gezeigte vorteilhafte Möglichkeit im Rahmen der erfindungsgemäßen Lösung besteht darin, dass der Stent 80 bedarfsweise auch durch einen zusätzlichen Hilfsstent 87 mit einer Seitöffnung 88 implantierbar ist.

Die in den Figuren 35 und 36 dargestellte Katheter-Stent-Vorrichtung ist besonders für Stenosen an Arterien-Abgängen geeignet, z. B. auch für die Stent-Therapie von Bifurkationen, insbesondere an den Koronararterien. Die beschriebene Katheter-Stent-Vorrichtung kann somit auch durch die Seitöffnung 88 eines anderen Hilfsstents 87 geschoben werden und somit eine Gefäßabzweigung vollkommen abdecken.

Die Figuren 37a, b und c zeigen weitere mögliche Ausgestaltungen, insbesondere des proximalen Hüllkatheterabschnitts 84 des Stents 80, in einer Draufsicht auf den Stent 80 aus proximaler Richtung.

Wie bereits erwähnt, gelingt die Aufdehnung des proximalen Stentabschnitts 81 zu einem Ringflansch 83 in erster Linie dadurch, dass radial nach außenweisende Stentstreben 89 zur Ausbildung dieses proximalen Stentabschnitts 81 vorgesehen sind. Dabei zeigen die Figuren 37 a bis c unterschiedliche Ausbildungen, insbesondere dieser Stentstreben 89, die in Abhängigkeit von dem jeweils zu behandelnden Körpergefäß wählbar sind.

Gemäß der Darstellung in Figur 37a erweitern sich die Stentstreben 89 zu einer trichterförmigen Rosette, die bestimmungsgemäß nach entsprechend distalen Vorschub des Stents 80 insgesamt an der Wandung des Hauptgefäßes anliegt.

Alternativ können an den distalen Stentabschnitt 82 zur Ausbildung des proximalen Stentabschnitts 81, aber auch nur einzelne, nicht miteinander verbundene Stentstreben 89 angeordnet sein, da diese bereits zur Ausbildung eines Anschlags an der Gefäßwandung des jeweiligen Hauptgefäßes ausreichen. Dabei kann insbesondere bei den Stentstreben 89 auf die Situation am Implantationsort vorteilhaft eingegangen werden. So ist es beispielsweise denkbar, dass gemäß der Darstellung in Figur 37c ein Nebengefäß 91 in einem spitzen Winkel von einem Hauptgefäß 90 abzweigt, so dass es bei einer derartigen Lösung vorteilhaft erscheint, dies auch bei der Ausbildung der Stentstreben 89 durch deren unterschiedliche Länge zu berücksichtigen.

Figur 38 zeigt eine weitere Variante der erfindungsgemäßen Katheter-Stent-Vorrichtung in einem Querschnitt.

In dieser Ausführung ist in dem Zentralkanal 51 zur Durchführung des Führungsdrahtes 52 zusätzlich ein separater Spülkanal 100 angeordnet. Der separate Spülkanal 100 wird proximalwärts bestimmungsgemäß mit einem Seitloch 101 mit einem geeigneten Fluid beschickt. Auch der Zentralkanal 51 weist ein proximalseitig angeformtes Seitloch 101 zur Einführung und Durchführung des Führungsdrahtes 52 auf, das im Einführungsbereich mit einer Abschlussdichtung 103 gesichert ist. Die Katheter-Stent-Vorrichtung ist ansonsten in herkömmlicherweise mit einer Distalspitze 53 versehen. Auch bei dieser Ausführung umschließt ein Hüllkatheter 54 einen zu Zwecken der Implantation zunächst komprimierten Stent 56, der in einem Stent-Bett 55 des Hauptkatheters 50 aufgenommen ist. Zusätzlich umschließt der Hüllkatheter 54 auch hier eine gegenüber dem Stent-Bett 55 abgedichtete Druckkammer 60.

Bevor die vorstehend, ihrem Aufbau nach kursorisch erläuterte Katheter-Stent-Vorrichtung in den Patientenkörper eingeführt wird, ist auch hier eine Spülung der Vorrichtung insgesamt mit einer geeigneten Spülflüssigkeit, etwa einer Kochsalzlösung vorgesehen. Hierzu wird über das eine Seitloch 101 der Spülkanal 100 mit der Strömungsflüssigkeit beaufschlagt, wobei durch eine entsprechende Spülöffnung 104 des Spelkanals 100 die Spülflüssigkeit bestimmungsgemäß in die Druckkammer 60 eindringt und durch eine Auslassöffnung 105 der Druckkammer 60, die in den Zentralkanal 51 mündet, gelangt die Spülflüssigkeit von dort sowohl in Richtung des Stent-Betts 55 und spült über eine entsprechende Stent-Bettspülöffnung 64 auch den Hüllkatheter 54 umschlossenen Stent-Bettbereich. Dabei tritt die Spülflüssigkeit durch den Stent-Bettbereich in distaler Richtung durch und strömt durch eine weitere Auslassöffnung 106 nach außen weg. Wie bereits vorstehend des Öfteren ausgeführt, wird bei der Spülung vor der Implantation die Katheter-Stent-Vorrichtung distalseitig einfach mit dem Finger des Operateurs verschlossen und von der Proximalseite die Spülflüssigkeit, wie oben erwähnt, eingeführt. Schließlich strömt die Spülflüssigkeit bestimmungsgemäß durch den Zentralkanal 51 auch zur Proximalseite zurück, wobei zum Zeitpunkt des Austritts von Spülflüssigkeit durch das proximale Seitloch 102 des Zentralkanals 51 zur Einführung des Führungsdrahts 52 ein sicheres Signal für den Operateur gegeben ist, dass nun die Katheter-Stent-Vorrichtung vollständig evakuiert ist.

Nun kann die Katheter-Stent-Vorrichtung über den Führungsdraht 52 bestimmungsgemäß in den Patientenkörper eingebracht werden, wobei dann im Weiteren der bereits an anderer Stelle erläuterte innere Ringwulst 66 an dem eingeführten Führungsdraht 52 anliegt und die Druckkammer 60 strömungstechnisch von dem Bereich des Stent-Betts 55 abtrennt. Sobald die Katheter-Stent-Vorrichtung ihre bestimmungsgemäße Lage erreicht hat, kann also auch hier durch entsprechende Beaufschlagung mit einem geeigneten Druckmedium die Druckkammer 60 über den separaten Spülkanal 100 druckbeaufschlagt werden, wobei auch in diesem Fall die Spülflüssigkeit durch den Zentralkanal 51 abfließen kann. Durch Beaufschlagung der Druckkammer 60 mit der Spülflüssigkeit wird jedenfalls auch hier die Druckkammer 60 expandiert, mithin der Hüllkatheter 54 in proximaler Richtung zurückgeschoben und der in dem Stent-Bett 55 aufgenommene, zunächst noch komprimierte Stent 56 bestimmungsgemäß freigesetzt.

Dabei ist es durchaus denkbar, dass bei bestimmungsgemäßer Positionierung der in dieser Figur dargestellten Katheter-Stent-Vorrichtung der separate Spülkanal über sein Seitloch 101 außerhalb des Patientenkörpers mündet, während der Zentralkanal 51 mit dem anderen Seitloch 102 im Patientenkörper mündet.

In Figur 38 ist somit ein doppellumiger Katheter gezeigt, mit dem eine Katheter-Stent-Vorrichtung implantierbar ist, bei der ein Hüllkatheter mit einer Spülflüssigkeit hydraulisch relativ zu einem selbstexpandierenden Stent derart bewegt wird, dass der selbstexpandierende Stent am Implantationsort bestimmungsgemäß freigegeben wird.

### BEZUGSZEICHENLISTE

- 1: Katheter
- 1': proximaler Anteil des Katheters
- 1": distale Öffnung des Katheters
- 2: Stent
- 2': Spiralstent
- 2": Slotted-tube-Stent
- 2"': Kugelkopf des Spiralstents
- 3: Körperhohlorgan oder -gefäß
- 4: Kolben
- 4': Distaler Kolben
- 4": kolbenförmige Erweiterung
- 4" ': Schieber des Kolbens
- 5: Träger
- 6: Seitloch im Katheter 1
- 7: Führungsdraht
- 7': zentraler Kanal zur Aufnahme des Führungsdrahtes
- 7 ": Hohldraht
- 8: Dichtung
- 8': hämostatisches Ventil
- 9: Polymer
- 10: Längsschlitz des Katheter
- 11: Stentträger
- 11': Stent-Bett
- 11": Kolbenteil des Träger
- 11''': Träger-Seitloch
- 12: Katheterschleuse
- 13: Pumpe
- 14: Injektionsspritze zur Spülung des Systems
- 14': Ansatzstück für Injektionsspritze bzw. Pumpe
- 15: doppellumiger Katheter
- 15': enges Lumen
- 15'': weites Lumen
- 16: Druckkammer
- 17: Verschlusshahn
- 18: Plattform
- 18': Plattformhülle
- 18": Kolben der Plattform
- 19: Querfalten
- 20: Halterohr für Stentträger
- 21: Stabilisationsrohr
- 22: Pusher-Katheter
- 30: Stentkammer
- 31: umlaufende Kante
- 32: Innenkatheter
- 33: Muffe
- 34: Spülloch
- 35: Spülspalt
- 36: Anschlagmuffe
- 37: Druckkolben
- 38: trichterförmige Erweiterung
- 50: Hauptkatheter
- 51: Zentralkanal
- 52: Führungsdraht
- 53: Distalspitze
- 54: Hüllkatheter
- 55: Stent-Bett
- 56: Stent
- 57: Außenring
- 58: Dichtungsring
- 60: Druckkammer
- 61: weitere Ringdichtung
- 62: Ringkolben
- 63: Druckfeder
- 64: Stent-Bettspülöffnung
- 65: Druckkammerspülöffnung
- 66: innerer Ringwulst
- 70: zweite Druckkammer
- 71: weitere Druckkammerspülöffnung
- 72, 72': weitere Ringdichtungen
- 80: Stent
- 81: proximaler Stentabschnitt
- 82: distaler Stentabschnitt
- 83: Ringflansch
- 84: proximaler Hüllkatheterabschnitt
- 85: distaler Hüllkatheterabschnitt
- 86: Katheterverschluss
- 87: Hilfskatheter
- 88: Seitöffnung
- 90: Hauptgefäß
- 91: Nebengefäß
- 100: Spülkanal
- 101: ein Seitloch
- 102: anderes Seitloch
- 103: Abschlussdichtung
- 104: Spülöffnung
- 105: Auslassöffnung
- 106: weitere Auslassöffnung

## Patentansprüche

1. Katheter-Stent-Vorrichtung nach dem Patent DE 10 2005 008 682 zur Applikation und Implantation eines Stents (2) zur Behandlung von Körpergefäßen oder Körperhohlorganen, **dadurch gekennzeichnet, dass** der Stent (2) und ein den Stent (2) umgebender Katheter (1) mittels einer Druckdifferenz zwischen dem proximalen - außerhalb des Körpers gelegenen - und dem distalen - im Körper gelegenen - Ende des Katheters (1) herrschenden Druckdifferenz in Relation zueinander beweglich sind, wobei diese Druckdifferenz mittels eines Druckmediums, bestehend aus einer Flüssigkeit oder aus einem Gas, aufgebaut wird und hierdurch der Stent (2) zielgerecht implantierbar ist.

2. Katheter-Stent-Vorrichtung nach Anspruch 1, **dadurch gekennzeichnet, dass** der Stent (2) mittels eines Kolben (4), dessen Umfang dem Lumen des Katheters (1) angepasst ist, freisetzbar ist.

3. Katheter-Stent-Vorrichtung nach Anspruch 1 oder 2, bei der zur Freisetzung des Stents (2) in Form eines langgestreckten fadenförmigen elastischen Drahtfilaments, vorzugsweise in Form eines Spiralstents (2') oder eines Mäander-Stents, das Drahtfilament des Stents (2) als Kolben wirkt und der Stent (2) hierdurch mittels der Druckdifferenz durch den Katheter (1) in ein tubuläres Körperhohlorgan (3) oder -gefäß befördert wird und der ebendort freigesetzte Stent (2) aufgrund der Elastizität des Drahtfilaments ein schlauchförmigen Gebilde ausformt.

4. Katheter-Stent-Vorrichtung nach einem der vorhergehenden Ansprüche, **dadurch gekennzeichnet, dass** mittels eines Druckmediums Druck auf einen röhrenförmigen selbstexpandierenden, aber noch nicht expandierten Stent (2) aufbringbar ist und dieser Stent (2) hierdurch aus den ihn umgebenden Katheter (2) am Implantationsort freisetzbar ist

5. Katheter-Stent-Vorrichtung nach Anspruch 4, **dadurch gekennzeichnet, dass** die Druckdifferenz im Katheter direkt auf das distale Ende des frei zu setzenden Stents (2) wirkt, das hierzu in Form eines distalen Kolbens(4') ausgebildet ist.

6. Katheter-Stent-Vorrichtung gemäß Anspruch 5, bei der der Stent (2) zusätzlich am proximalen Stentende einen oder mehrere in Reihenform angeordnete kolbenförmige Erweiterungen (4") aufweist und das Druckmedium auch auf diese kolbenförmigen Erweiterungen (4'') des Stents (2) einwirkt und hierdurch den Stent (2) aus dem umgebenden Katheter (1) hinaus treibt, wobei nur der distalseitig, vorne auf dem Stent (2) angebrachte distale Kolben (4) das Lumen im Katheter (1) vollständig ausfüllt, während die nachfolgend, vorzugsweise am proximalen Stentende, angeordneten kolbenförmigen Erweiterungen (4'') allenfalls ein geringes Vorbeiströmen des Druckmediums erlauben, so dass nach Heraustreten des vordersten distalen Kolbens (4) die nachfolgenden Stentteile durch die weiter hinten angeordneten kolbenförmigen Erweiterungen (4'') ausgeschoben werden.

7. Katheter Stent-Vorrichtung nach Anspruch 5 oder 6, **dadurch gekennzeichnet, dass** der distale Kolben (4) und/oder die kolbenförmige/n Erweiterung/en (4'') des Stents (2) aus einem in den Körpersäften löslichem Material ausgebildet ist/sind.

8. Katheter-Stent-Vorrichtung nach einem der vorhergehenden Ansprüche, mit einem im Katheter beweglichen separaten Kolben (4), wobei mit diesem Kolben (4)der zu platzierende Stent (2) mittels des Druckmediums durch den Katheter (1) treibbar und letztlich aus dem Katheter (1) ausschiebbar ist.

9. Katheter-Stent-Vorrichtung nach Anspruch 8, **dadurch gekennzeichnet, dass** der bewegliche Kolben (4) einen zentralen Kanal (7') aufweist, in dem ein Führungsdraht (7) aufgenommen ist.

10. Katheter Stent-Vorrichtung nach Anspruch 8 oder 9, **dadurch gekennzeichnet, dass** die Wand des den beweglichen Kolben (4) umgebenden Katheters (1) ein Seitloch (6) aufweist, welches nach Vorbeigleiten des Kolbens (4) in distaler Richtung und nach abgeschlossener Freisetzung des Stents (2) derart eröffnet ist, dass das unter Druck stehende Druckmedium durch dieses Seitlich(6)entweichen kann und hierdurch einen weiterer Vortrieb des Kolbens (4), insbesondere dessen unerwünschte Freisetzung aus dem Katheter (1), in das zu behandelnde Körpergefäß oder -hohlorgan (3) verhindert ist.

11. Katheter-Stent-Vorrichtung nach einem der vorhergehenden Ansprüche, **dadurch gekennzeichnet, dass** die Katheter-Stent-Vorrichtung ein für das Druckmedium abgeschlossenes Drucksystem darstellt, in dem ein selbstexpandierender Stent (2) auf einem röhrenförmigen Träger (11) montiert ist, der ein den Stent (2) aufnehmendes Stent-Bett (11') aufweist, dessen vorderes Ende zu einer Spitze geformt ist und dessen hinteres Ende mit einem Kolbenteil (11'') versehen ist und der mit einem Kanal für einen Führungsdraht (7) versehen ist, der im distalen Bereich des Trägers (11) zentral verläuft und dann im proximal angeordneten Kolbenteil (11'') schräg nach außen verläuft und schließlich in einem Träger-Seitloch (11"') mündet, wobei der Führungsdraht (7) durch das Kolbenteil (11'') verläuft und dann durch das Träger-Seitloch (11"') hinaustritt, wobei durch das Herausdrücken des Trägers (11) der Stent (2) aus dem Katheter (1) freigesetzt wird und hierbei der Kolbenteil (11'') des Trägers (11) im Katheter (1) verbleibt, da ein weiteres Hinausgleiten des Kolbenteils (11'') aus dem Katheter (1) durch Sperrung des aus dem Kolben-Seitloch (11"') seitlich austretenden Führungsdraht (7) verhindert ist.

12. Katheter-Stent-Vorrichtung nach einem der vorhergehenden Ansprüche, **dadurch gekennzeichnet, dass** die Stirnfläche eines Pumpkolbens einer Pumpe (13) gleich dimensioniert ist, wie die zur Druckbeaufschlagung zur Verfügung stehende Stirnfläche des im Katheter (1) befindlichen Kolbens (4).

13. Katheter-Stent-Vorrichtung nach einem der Ansprüche 1 bis 11, **dadurch gekennzeichnet, dass** die Stirnfläche des Pumpkolbens der Pumpe (13) eine andere Größe hat als die Stirnfläche des zur Druckbeaufschlagung zur Verfügung stehenden Kolbens (4) im Katheter (1).

14. Katheter-Stent-Vorrichtung nach einem der vorhergehenden Ansprüche 11 bis 13, **dadurch gekennzeichnet, dass** der Schaft (15) des Katheters (1) zur Durchführung einer Spülung und zur Entlüftung der in sich abgeschlossenen Drucksystems zwei Lumina (15', 15'') aufweist.

15. Katheter-Stent-Vorrichtung nach Anspruch 14, **dadurch gekennzeichnet, dass** ein kleineres Lumen (15') der Lumina zusätzlich der Druckbeaufschlagung des Drucksystems und ein größeres Lumen (15'') der Lumina zusätzlich zur Aufnahme des Führungsdrahts (7) dient.

16. Katheter-Stent-Vorrichtung nach einem der vorgehenden Ansprüche, bei dem ein den Stent (2) tragender Stentträger nach Platzierung des Stents (2) im Körpergefäß oder -hohlorgan (3) in fester Position verbleibt und der Stent (2) **dadurch** freigesetzt wird, dass mittels entsprechender Druckbeaufschlagung der Kolben (4) zusammen mit dem den Stentträger (11) umgebenden Hüllkatheter zurückgeleitet.

17. Katheter-Stent-System nach Anspruch 16, bei dem der den Stent (2) umgebende Hüllkatheter bei noch nicht vollständig expandiertem Stent (2) wieder über den Stent (2) hinweg in die Ausgangsposition zurückbeweglich ist.

18. Katheter-Stent-System nach Anspruch 16 oder 17, bei dem das Druckmedium in einer Druckkammer (16) des Drucksystems und den mit ihr verbundenen Kanälen vor Gebrauch eingefüllt wird und abgeschlossen ist.

19. Katheter-Stent-Vorrichtung nach Anspruch 1, bei der der Stent mittels eines Hauptkatheters (50) zum Zwecke der Implantation über einen Führungsdraht (52) in den Körper einführbar ist, implantierbar ist, wobei der selbstexpandierende Stent (56) hierzu in einem distalseitig angeordneten Stent-Bett (55) des Hauptkatheters (50) derart aufgenommen ist, dass der zum Zwecke der Implantation zunächst komprimierte Stent (56) den Hauptkatheter (50) im Bereich dieses Stent-Betts (55) konzentrisch umschließt und hierbei der komprimierte Stent (56) sowie der Hauptkatheter (50) von einem Hüllkatheter (54) insgesamt konzentrisch umschlossen sind, wobei die Stenthülle zusätzlich eine sich in proximaler Richtung an das Stent-Bett (55) anschließende Druckkammer (60) umschließt.

20. Katheter-Stent-Vorrichtung nach Anspruch 19, **dadurch gekennzeichnet, dass** die Druckkammer (60) gegenüber dem Stent-Bett (55), vorzugsweise mittels eines Dichtungsrings (58), distalseitig abgeschlossen ist.

21. Katheter-Stent-Vorrichtung nach Anspruch 19 oder 20, **dadurch gekennzeichnet, dass** zwischen der Druckkammer (60) und dem Stent-Bett (55) ein feststehender Ringkolben (62) in der Katheterhülle angeordnet ist.

22. Katheter-Stent-Vorrichtung nach Anspruch 21, **dadurch gekennzeichnet, dass** die Druckkammer (60) proximalseitig von wenigstens einem weiteren längsbeweglichen Ringkolben (62) und einer weiteren Dichtung proximalseitig abgeschlossen ist.

23. Katheter-Stent-Vorrichtung nach einem der Ansprüche 20 bis 22, **dadurch gekennzeichnet, dass** sowohl im Bereich des Stent-Betts (55), wie auch im Bereich der Druckkammer (60) eine Spülöffnung in der Wand des Hauptkatheters (50) angeordnet ist, die mit dem innen liegenden, konzentrisch angeordneten Zentralkanal (51) zur Durchführung des Führungsdrahts (52) strömungsverbunden sind.

24. Katheter-Stent-Vorrichtung nach Anspruch 23, **dadurch gekennzeichnet, dass** zwischen der Stent-Bettspülöffnung (64) und der Druckkammerspülöffnung (32) des Hüllkatheters (54) ein den Führungsdraht (52) konzentrisch umschließender dichtender Ringwulst (66) an die Innenwandung des Hüllkatheters (54) angeformt ist.

25. Katheter-Stent-Vorrichtung nach einem der Ansprüche 21 bis 24, **dadurch gekennzeichnet, dass** die Druckkammer (60) mittels eines Druckmediums von der Distalseite her derart druckbeaufschlagbar ist, dass in Folge der Druckbeaufschlagung der Hüllkatheter (54) relativ zum Hauptkatheter (50) und somit auch zum Stent-Bett (55) bis zur Freigabe des in dem Stent-Bett (55) aufgenommenen, komprimierten Stents (56) längsbeweglich ist.

26. Katheter-Stent-Vorrichtung nach einem der Ansprüche 21 bis 25, **dadurch gekennzeichnet, dass** in der vom Hüllkatheter (54) konzentrisch umschlossenen Druckkammer (60) zusätzlich wenigstens eine auf dem proximalseitigen, weiteren, längsbeweglichen Ringkolben (62) einwirkenden wenigstens einen Druckfeder (63) angeordnet ist.

27. Katheter-Stent-Vorrichtung nach einem der vorhergehenden Ansprüche, **dadurch gekennzeichnet, dass** die Katheter-Stent-Vorrichtung insgesamt vor der Implantation distalseitig verschließbar ist und, vorzugsweise mittels einer Kochsalzlösung, zum Zwecke der Luftevakuierung, insbesondere im Bereich des Stent-Betts (55) und der Druckkammer (60) von der Distalseite des Katheters (1) her spülbar ist.

28. Katheter-Stent-Vorrichtung nach einem der vorhergehenden Ansprüche, **dadurch gekennzeichnet, dass** nach erfolgter Implantation der Katheter-Stent-Vorrichtung in deren lagerichtiger Positionierung die Druckkammer (60) mittels eines distalseitig eingepumpten Druckmediums derart druckbeaufschlagbar ist, dass die Katheterhülle in Folge der insoweit gemeinsam einwirkenden Druckkräfte des Druckmediums und der wenigstens einen Druckfeder (63) auf den weiteren proximalseitigen Ringkolben (62) in proximaler Richtung zurückschieben und hierdurch der in dem Stent-Bett (55) aufgenommene, komprimierte Stent (56) am Implantationsort lagerichtig freigesetzt wird und ebendort expandiert.

29. Katheter-Stent-Vorrichtung nach einem der vorhergehenden Ansprüche, **dadurch gekennzeichnet, dass** die das Stent-Bett (55) und die Druckkammer (60) konzentrisch umschließende Hüllkatheter (54) zweiteilig mit einem distalseitigen und einem proximalseitigen Hüllkatheterabschnitt (84) ausgebildet ist und auch der in dem Stent-Bett (55) aufgenommene, selbstexpandierende Stent (56) zwei unterscheidbare Abschnitte, nämlich einen distalen Stentabschnitt (82) und einen proximalen Stentabschnitt (81) aufweist, wobei der distale Stentabschnitt (82) sich am Implantationsort bestimmungsgemäß von einem kleineren Lumen auf ein größeres Lumen erweitert, während der proximalseitig angeordnete Stentabschnitt (81) sich zur Ausbildung eines Ringflanschs (83) bestimmungsgemäß erweitert.

30. Katheter-Stent-Vorrichtung nach Anspruch 29, **dadurch gekennzeichnet, dass** der den Führungsdraht (52) umschließende Hauptkatheter (50) mit einem distalseitigen Verschluss (86) verschlossen ist.

31. Katheter-Stent-Vorrichtung nach Anspruch 29 oder 30, **dadurch gekennzeichnet, dass** die von dem Hüllkatheter (54) umschlossene Druckkammer (60) distalseitig und das ebenfalls vom Hüllkatheter (54) umschlossene Stent-Bett (55) sich hieran proximalseitig anschließt.

32. Katheter-Stent-Vorrichtung nach einem der Ansprüche 29 bis 31, **dadurch gekennzeichnet, dass** nach lagerichtiger Positionierung der Katheter-Stent-Vorrichtung am Implantationsort der proximalseitige Hüllkatheterabschnitt (84) zurückziehbar ist und hierdurch der zunächst komprimierte Proximalabschnitt (81) des selbstexpandierenden Stents (80) freigegeben wird, der sich dann bestimmungsgemäß am Implantationsort ringflanschartig erweitert.

33. Kather-Stent-Vorrichtung nach einem der Ansprüche 29 bis 32, **dadurch gekennzeichnet**, der selbstexpandierende proximalseitige Stentabschnitt (81) zur Eröffnung des Ringflansches ausschließlich aus bei deren Freisetzung radial nach außen strebenden Stentstreben besteht.

34. Katheter-Stent-Vorrichtung, nach einem der Ansprüche 29 bis 33, **dadurch gekennzeichnet, dass** die distalseitig angeordnete Druckkammer (60) durch eine entsprechende Öffnung zum Hüllkatheter (54) mit einem Druckmedium druckbeaufschlagbar ist und hierdurch der distalseitige Hüllkatheterabschnitt (85) in distaler Richtung relativ zu dem teilweise noch im Stent-Bett (55) aufgenommenen Stent (80) längsbeweglich ist und hierdurch nun auch der distalseitige Stentabschnitt (82) freisetzbar und am Implantationsort expandierbar ist.

35. Katheter-Stent-Vorrichtung nach einem der Ansprüche 28 bis 34, **dadurch gekennzeichnet, dass** zum Abschluss des Implantationsvorganges nach erfolgter Expansion beider Stentabschnitte (81, 82) der Hüllkatheter (54) mit dem Führungsdraht (52) und dem Hauptkatheter (50) durch den erweiterten Stent (80) abziehbar ist.

36. Katheter-Stent-Vorrichtung nach einem der vorhergehenden Ansprüche, **dadurch gekennzeichnet, dass** in dem Hüllkatheter (54) wenigstens eine weitere druckbeaufschlagbare Druckkammer (70) in Reihenschaltung zu der ersten Druckkammer (60) angeordnet ist, die in Verbindung mit der ersten Druckkammer (60) mittelbar auf den längsbeweglichen Hüllkathether (54) einwirkt.

37. Katheter-Stent-Vorrichtung nach einem der vorhergehenden Ansprüche, **dadurch gekennzeichnet, dass** in den Zentralkanal (51) des Hauptkatheters (50) ein separater abgeschlossener Spülkanal (100) angeordnet ist, der über entsprechende Öffnungen jeweils mit der oder den Druckkammern (60,70) strömungsverbunden ist, wobei die Druckkammern (60, 70), jeweils mit dem Zentralkanal über eine Rückströmöffnung strömungsverbunden sind, die bei eingeführten Führungsdraht 52 verschlossen ist.

38. Katheter-Stent-Vorrichtung nach Anspruch 37, **dadurch gekennzeichnet, dass** der Spülkanal (100) und/oder der Zentralkanal (51) jeweils über ein Seitloch (101, 102) des Hauptkatheters (50) proximalwärts versorgbar sind.

39. Katheter-Stent-Vorrichtung nach Anspruch 38, **dadurch gekennzeichnet, dass** das Seitloch (102) des Zentralkanals (51) derart entlang der Längserstreckung des Hauptkatheters (50) angeordnet ist, dass bei lagerichtiger Positionierung des Hauptkatheters (50) in einem Patientenkörper dieses Seitloch (102) innerhalb des Patientenkörpers positioniert ist.
